# EUROPEAN PATENT SPECIFICATION

(11) **EP 2 853 255 B1**
(45) Date of publication and mention of the grant of the patent: **28.11.2018**
(21) Application number: 14185253.3
(22) Date of filing: 18.09.2014
(51) Int. Cl.: A61K 8/37, A61K 8/49, A61Q 17/04, A61Q 19/00, A61K 8/81, A61K 8/06

(54) **Method of formulating a personal care product with substantially no whitening effect when applied on wet skin and compositions thereof**
Verfahren zur Formulierung eines Körperpflegeprodukts mit nahezu keiner Bleichungswirkung beim Auftragen auf feuchte Haut sowie Zusammensetzungen damit
Procédé de formulation d'un produit de soins personnels avec pratiquement aucun effet de blanchiment lorsquoeil est appliqué sur une peau humide et compositions associées

(30) Priority: 27.09.2013 US 201361883848 P; 28.03.2014 US 201414229590
(43) Date of publication of application: 01.04.2015
(73) Proprietor: HallStar Beauty and Personal Care Innovations Company, Chicago, IL 60606 (US)
(72) Inventor: Bonda, Craig A., Winfield, IL Illinois 60190 (US); Zhang, Qing Jean, Hickory Hills, IL Illinois 60457 (US)
(74) Representative: Barker Brettell LLP

(56) References cited:
- EP-A1- 0 998 910
- WO-A1-2007/144670
- US-A- 5 833 951
- US-A1- 2002 146 443
- US-B2- 8 236 287
- DATABASE GNPD [Online] MINTEL; April 2012 (2012-04), "Vitamin E sunscreen SPF 30", XP002734764, Database accession no. 1764119
- DATABASE GNPD [Online] MINTEL; December 2012 (2012-12), "Sunscreen lotion SPF 20", XP002734765, Database accession no. 1936838
- DATABASE GNPD [Online] MINTEL; June 2013 (2013-06), "Day protection sunscreen lotion", XP002734766, Database accession no. 2086609

## Description

### FIELD OF THE DISCLOSURE

The present disclosure relates to a method of formulating a personal care product composition (e.g. sunscreen) that may remain stable when exposed to high and low temperatures for prolonged periods and, when applied to wet skin, may be substantially devoid of any whitening effect while not compromising its other functions (e.g. protecting the skin against solar UV radiation).

### BACKGROUND OF THE DISCLOSURE

Personal care products often contain an active protective component. For example, sunscreen products may comprise UV-absorbing or scattering compounds (collectively "sunscreen actives" or "UV filters") formulated into a cosmetically suitable vehicle for topical application to the skin. Ideally, once applied to and spread on the skin, the active protective component (e.g. UV filters or other lipophilic ingredients contained in the composition) may form a thin continuous film on the skin which provides an effective, protective barrier against environmental insults (e.g. UV radiation). Such products have proven successful for many years at protecting skin, especially when applied to dry skin. However, many personal care products (e.g. sunscreen) when applied to wet skin tend to smear or form an incomplete film and exhibit a persistent, unpleasant white appearance. Such products may not provide the expected protection, with potentially negative consequences (e.g. sunburn) for the wearer. Some personal care products may be formulated as emulsions wherein the bulk phase is comprised of organic or silicone oils, and the discontinuous phase is comprised of water and/or water-soluble components. Even if these products, when applied to wet skin, adequately perform their protective function, they suffer from an unpleasant greasy feel and persistent whitening that requires excessive rubbing to return the skin's surface to an acceptable appearance. Recently, some products have appeared that claim to be formulated specifically for application to wet skin with little or none of the deficits of more traditional personal care products. For example, U.S. Patent No. 8,236,287 issued to Singleton teaches a sunscreen composition comprising a continuous water phase, a discontinuous oil phase dispersed in the water phase, about 10% or more by weight of an organic UV filter, a water-insoluble C2-C8 liquid silicone, a branched fatty acid ester of a polyprotic carboxylic acid, and about 2% by weight of a mineral particulate that bears a starch coating on its surface. Said composition is reputed to substantially maintain its UV protective property after it is applied to wet skin compared to its UV protective property when applied to dry skin. Further, the composition is said to be capable of providing "little to no whitening" when applied to wet skin. However, embodiments of Singleton in the form of commercially available products - Neutrogena® Wet Skin SPF 45+ sunscreen and Neutrogena® Wet Skin Kids SPF 45+ sunscreen - failed to apply clear to wet skin.

US 5,833,951 discloses cosmetic and dermatological formulations of the oil-in water type, processes for their preparation and their use for cosmetic and medical purposes.

EP 0998910 discloses a personal care product having acontinuous water phase, a discontinuous oil phase, preferable free from emulsifiers and comprising a glyceride wax.

Database GNPD, Mintel June 2013 (2013-06), 'Data protection sunscreen lotions' Dabatase Accesssion No. 2086609 discloses a sunscreen comprising a non-emulsifying carbomer.

### SUMMARY

Consequently, a need exists for personal care products (e.g. sunscreen) that apply clear to wet skin (e.g. by swiping, rubbing, smoothing, massaging, coating, or otherwise working the composition on the skin) and remain clear while also providing the expected level of protection to the wearer.

The present disclosure relates, in some embodiments, to methods and compositions for personal care products that become transparent upon application to wet skin, remain clear, are aesthetically pleasant, maintain stability when exposed to high and low temperatures over prolonged periods, and/or have the ability to protect the wearer's skin from damaging ultraviolet radiation.

The present disclosure relates to methods of formulating personal care products (e.g. sunscreen) and compositions thereof. Personal care product compositions may comprise a water phase into which an oil phase comprising organic UV filters, suitable organic and/or silicone solvents, emollients, and oil soluble polymers may be dispersed. In some embodiments, a dispersion of oil may be aided by a low HLB emulsifier that may be a liquid at room temperature and present at 0.1-1% by weight. In some embodiments, a water phase may be gelled by neutralizing one or more polymers of acrylic acid cross-linked with allyl sucrose or allyl pentaerythritol, hereinafter referred to as a "Carbomer Homopolymer," or a non-emulsifying grade of Carbomer Homopolymer that may also contain a block copolymer of polyethylene glycol and a long chain alkyl acid ester, hereinafter referred to as a "Carbomer Interpolymer," or a non-emulsifying grade of a copolymer of acrylic acid and a long chain alkyl methacrylate cross-linked with allyl ethers of polyalcohols, hereinafter referred to as a "Carbomer Copolymer;" or a combination of Carbomer Homopolymer, Carbomer Interpolymer, and/or Carbomer Copolymer wherein the total weight percentage of carbomer homopolymer, carbomer interpolymer, and/or carbomer copolymer may be present in a range of about 0.2-0.5% of the composition. In some embodiments, the composition may contain an SPF boosting compound, for example, Styrene Acrylates copolymer, which may be available as SUNSPHERES from Dow Chemical Company at a weight percentage of the composition in a range from 0-2%. Some embodiments of the present disclosure may apply clear to wet skin (e.g., a composition may become transparent with repeated swiping rubbing, smoothing, massaging, coating, or otherwise working the composition on the skin), such that the skin's natural color and appearance are visible. In some embodiments, a personal care product composition may apply clear without compromising the photoprotective properties of the composition.

In some embodiments, the present disclosure relates to methods of formulating an oil-in-water personal care product composition that is stable when exposed to temperatures of about 60°C and/or about 5°C for a period of one week or more. For example, a method may comprise (a) providing separately an oil phase and a water phase, (b) dispersing one or more carbomer polymers in either the oil phase or the water phase, (c) optionally dispersing a hydrophilic-lipophilic balance (HLB) emulsifying compound in the oil phase, wherein the HLB emulsifying compound is a liquid at room temperature and has a HLB less than about 5; (d) combining the oil phase with the water phase to form a pre-emulsion, (e) adding a neutralizing base to the pre-emulsion to form a personal care product emulsion, (f) adding water to the personal care product emulsion, and (g) mixing the personal care product emulsion to form a homogenous composition, wherein the homogenous composition comprises from about 0.1% to about 1% by weight of the HLB emulsifying compound, a stabilizing amount of the one or more carbomer polymers, each carbomer polymer selected from the group consisting of a carbomer copolymer, a carbomer interpolymer, and a carbomer homopolymer, wherein none of the one or more carbomer polymers are emulsifying grade and wherein the total carbomer polymer concentration is less than 0.5% by weight of the, and, wherein the homogenous composition is free of any emulsifying compound..

The present disclosure relates, according to some embodiments, to methods of formulating a personal care product (e.g. sunscreen) composition that is stable when exposed to temperatures of about 60°C and/or about 5°C for a period of one week or more and that applies clear to wet skin and remains clear. A method may comprise, for example, combining a discontinuous oil phase with a hydrophilic-lipophilic balance (HLB) emulsifying compound to form an oil phase mixture, wherein the HLB emulsifying compound is a liquid at room temperature and has a HLB less than about 5; heating and mixing the oil phase mixture; dispersing one or more non-emulsifying grade carbomer polymers in a continuous water phase to form a water phase mixture; combining and mixing the oil phase mixture and the water phase mixture to form a pre-emulsion; adding a neutralizing base to the pre-emulsion to form a personal care product emulsion; adding water (e.g., to replace water lost during processing) to the personal care product emulsion; and mixing the personal care product emulsion to form a homogenous composition, wherein the homogenous composition comprises: from about 0.1% to about 1% by weight of the HLB emulsifying compound, a stabilizing, non-whitening amount of one or more carbomer polymers selected from the group consisting of a carbomer copolymer, carbomer interpolymer, and carbomer homopolymer, wherein none of the one or more carbon polymers is emulsifying grade and wherein the total carbomer polymer concentration is less than 0.5% by weight of the composition, and wherein the homogenous composition is free of any other emulsifying compound.

A stable composition may, in some embodiments, maintain its integrity after prolonged exposure (e.g. one month) to high temperatures (e.g. about 60°C), and low temperatures (e.g. about 5°C). The present disclosure relates, in some embodiments, to methods for applying a personal care product composition to a wet human skin surface. A method for applying the personal care product composition to the wet human skin surface may comprise, for example, swiping, rubbing, smoothing, massaging, coating, or otherwise working the composition on the skin. In some examples, the applying continues for less than about 5 seconds and the personal care product composition is clear no later than at the conclusion of the applying. In some examples of the method for applying the personal care product composition, the composition is clear throughout the applying.

According to an aspect of the invention, there is provided a personal care product composition for topical application to human skin that applies clear to wet skin comprising:
a continuous water phase;
a discontinuous oil phase dispersed in the continuous water phase, the discontinuous oil phase optionally comprising one or more UV filters at a concentration of up to about 50 wt.% of the total composition;
a hydrophilic-lipophilic balance (HLB) emulsifying compound at a concentration of about 0.1 wt.% to about 1 wt.% of the total composition, wherein the HLB emulsifying compound is a liquid at room temperature and has a HLB less than about 5, the composition lacking any other emulsifying compound;
a neutralizing base; and
a stabilizing, non-whitening amount of the one or more carbomer polymers, each carbomer polymer selected from the group consisting of a carbomer copolymer, a carbomer interpolymer, and a carbomer homopolymer, wherein none of the one or more carbomer polymers are emulsifying grade and wherein the total carbomer polymer concentration is less than 0.5% by weight of the composition, and
wherein the low HLB emulsifying compound is sorbitan oleate or glyceryl oleate.

### BRIEF DESCRIPTION OF THE DRAWINGS

The file of this patent contains at least one drawing executed in color. Copies of this patent with color drawing(s) will be provided by the Patent and Trademark Office upon request and payment of the necessary fee.

Some embodiments of the disclosure may be understood by referring, in part, to the present disclosure and the accompanying drawings, wherein:
FIGURE 1 illustrates an application of water to skin according to a specific example embodiment of the disclosure;
FIGURE 2 illustrates an application of a personal care product composition to wet skin according to the specific example embodiment of FIG. 1;
FIGURE 3 illustrates an application of a personal care product after two swipes according to the specific example embodiment of FIG. 1-2;
FIGURE 4 illustrates an application of a personal care product after three swipes according to the specific example embodiment of FIG 1-3;
FIGURE 5 illustrates an application of a personal care product after four swipes according to the specific example embodiment of FIG. 1-4;
FIGURE 6 illustrates an application of a personal care product after five swipes according to the specific example embodiment of FIG. 1-5;
FIGURE 7 illustrates an application of water to skin according to a specific example embodiment of the disclosure;
FIGURE 8 illustrates an application of a personal care product composition to wet skin according to the specific example embodiment of FIG. 7;
FIGURE 9 illustrates an application of a personal care product after one swipe according to the specific example embodiment of FIGURES 7-8;
FIGURE 10 illustrates an application of a personal care product after two swipes according to the specific example embodiment of FIGURES 7-9;
FIGURE 11 illustrates an application of a personal care product after three swipes according to the specific example embodiment of FIGURES 7-10; and
FIGURE 12 illustrates a completed application of a personal care product according to the specific example embodiment of FIGURES 7-11.

### DETAILED DESCRIPTION

According to some embodiments, a carbomer copolymer, a carbomer interpolymer, and/or a carbomer homopolymer may include the compendia names assigned to various cross-linked polymers of acrylic acid as set forth in the United States Pharmacopeia and National Formulary (USP 34-NF 29), United States Pharmacopeial Convention, Rockville, MD, 2010. The term carbomer copolymer may refer to a high molecular weight copolymer of acrylic acid and a long chain alkyl methacrylate cross-linked with allyl ethers of polyalcohols. Carbomer copolymers may be polymers of acrylic acid and C10-C30 alkyl acrylate crosslinked with allyl pentaerythritol. The term carbomer interpolymer may refer to a carbomer homopolyer or copolymer that contains a block copolymer of polyethylene glycol and a long chain alkyl acid ester. The term carbomer homopolyer may refer to a high molecular weight polymer of acrylic acid cross-linked with allyl ethers of polyalcohols. Carbomer homopolymers, previously dried, may not contain less than about 56 percent and not more than about 68 percent of carboxylic acid (-COOH) groups.

In some embodiments, Sun Protection Factor (SPF) may be the UV energy required to produce a minimal erythemal dose (MED) on protected skin (PS) divided by the UV energy required to produce a minimal erythemal dose on unprotected skin (US), which may also be defined by the following ratio: SPF value = MEDPS/MEDUS, where MEDPS is the minimal erythemal dose for protected skin after application of 2 milligrams per square centimeter of the sunscreen product, and MEDUS is the minimal erythemal dose for unprotected skin, i.e. skin to which no sunscreen product has been applied.

The UVA Protection Factor (UVA-PF) is the UVA (radiation with wavelengths from 320 to 400 nm) energy required to produce a minimal pigmentary dose (MPD) on protected skin (PS) divided by the UV energy required to produce a minimal pigmentary dose on unprotected skin (US), according to some embodiments, which may also be defined by the following ratio: UVA-PF value = MPDPS/MPDUS, where MPDPS is the minimal pigmentary dose for protected skin after application of 2 milligrams per square centimeter of the sunscreen product, and MPDUS is the minimal pigmentary dose for unprotected skin, i.e. skin to which no sunscreen product has been applied.

A material or composition may be cosmetically acceptable if it is suitable for application to human tissue (e.g., the skin) without undue toxicity, incompatibility, instability, irritation, allergic response, or the like, according to some embodiments. Percentages herein are percent by weight based on total concentration of the composition, oil or water phases.

A composition may be regarded to be stable or have stability where it maintains its integrity in form and function when subjected to high temperatures (e.g., about 60°C or higher) for an extended period of time (e.g., about one week or more) or when subjected to low temperatures (e.g., about 5°C or lower) for an extended period of time (e.g., about one week or more) according to some embodiments. A composition may maintain its integrity in form and/or shows no signs of instability such as discoloration, separation (e.g., oil appearing on the surface or the composition settling into layers), appearance of crystals, changes in room temperature viscosity, etc. By maintaining its integrity in function, it is meant that the composition, when returned to room temperature, may provide the same or similar levels of UV protection after exposure to high or low temperatures as before.

A composition, according to some embodiments, may be formulated to have any desired viscosity. For example, a composition have a viscosity that permits it to be applied to (e.g., easily applied to) and/or spread on (e.g., easily spread on) skin (e.g., wet skin). In some embodiments, a composition may have a viscosity (e.g., a Brookfield RVT viscosity) of about 300 cP to about 115,000 cP, about 4,000 cP to about 11,000 cP, about 29,400 cP to about 39,400 cP, about 30,500 cP to about 39,400 cP, about 1,000 cP to about 10,000 cP, about 10,000 cP to about 20,000 cP, about 20,000 cP to about 30,000 cP, about 30,000 cP to about 40,000 cP, about 40,000 cP to about 50,000 cP, about 50,000 cP to about 60,000 cP, about 60,000 cP to about 70,000 cP, about 70,000 cP to about 80,000 cP, about 80,000 cP to about 90,000 cP, about 90,000 cP to about 100,000 cP, and/or about 100,000 cP to about 110,000 cP. According to some embodiments, aqueous viscosity may be assayed (e.g., with respect to ranges disclosed herein) at a carbomer concentration of about 0.5 wt% mucilage at pH 7.5 and 20 rpm at 25□ C. Aqueous viscosity may be assayed according to USP 34-NF 29 in some embodiments.

In some embodiments, a whitening composition when applied to the skin and spread by a back-and-forth or circular motion of a finger for a few seconds may retain a whitish appearance that partially or completely obscures the skin's natural appearance and color. A composition may apply clear, quickly clarify, be clear, have excellent clarity and/or be non-whitening when applied to skin. According to some embodiments, a composition applies clear to wet skin if it is brought into contact with wet skin by limited swiping, rubbing, smoothing, massaging, coating, or otherwise working the composition on the skin, such that the skin's natural color and appearance are visible throughout and/or at the conclusion of application. Limited swiping, rubbing, smoothing, massaging, coating, or otherwise working the composition on the skin may include a few repetitions (e.g., from about 1 to about 5 swipes) and/or continued action (e.g., for about 2 to about 5 seconds). A composition may emerge from its container with a white appearance and, according to some embodiments, still apply clear to wet skin and/or still be non-whitening on wet skin so long as clarity is achieved while the skin is still wet. Wet skin may be damp with sweat or have residual water thereon, for example, immediately after being passed under a running faucet or immediately after being immersed in water, according to some embodiments.

According to another aspect of the invention, there is provided a method of applying a personal care product composition to a wet human skin surface having a natural appearance and color, the method comprising:
applying the personal care product composition to the wet human skin surface, the personal care product composition comprising:
a continuous water phase;
a discontinuous oil phase dispersed in the continuous water phase, the discontinuous oil phase comprising one or more UV filters at a concentration of at least about 5 wt.% of the total composition;
a hydrophilic-lipophilic balance (HLB) emulsifying compound at a concentration of about 0.1 wt.% to about 1 wt.% of the total composition, wherein the HLB emulsifying compound is a liquid at room temperature and has a HLB of less than about 5, the composition lacking any other emulsifying compound;
a neutralizing base; and
a stabilizing, non-whitening amount of the one or more carbomer polymers, each carbomer polymer selected from the group consisting of a carbomer copolymer, a carbomer interpolymer, and a carbomer homopolymer, wherein none of the one or more carbomer polymers are emulsifying grade and wherein the total carbomer polymer concentration is less than 0.5% by weight of the composition,
wherein
the applying comprises swiping, rubbing, smoothing, massaging, coating, or otherwise working the composition on the skin,
the applying continues for less than about 5 seconds, and
the personal care product composition is clear no later than at the conclusion of the applying, and
the skin with the applied personal care product composition is shielded from exposure to at least one wavelength of light relative to skin without the personal care product, and optionally or preferably wherein the personal care product composition is clear throughout the applying.

Compositions of the present disclosure may include a discontinuous oil phase and a continuous water phase. By "discontinuous oil phase," it is meant that a physically distinctive aggregation of intimately mixed organic, hydrophobic compounds that are stabilized within a water phase. Compounds that may be included in the oil phase include, for example, organic UV filters, suitable organic and/or silicone-based stabilizers (e.g., photostabilizers), solvents and emollients, film-forming polymers, SPF boosting compounds and polymers, and other hydrophobic compounds. In some embodiments, up to about 1% (w/w) of a low hydrophilic-lipophilic balance emulsifier may be included in a composition. The concentration of the oil phase may range, as a weight percent of the total composition, from about 10% to about 80%, such as from about 20% to about 60%, such as about 30% to about 50%.

A composition may have, according to some embodiments, a continuous water phase including a physically distinctive aggregation of water and other optional ingredients that are generally hydrophilic and intimately mixed therewith. Ingredients suitable for inclusion in the water phase include for example water, water-soluble preservatives, humectants, chelating agents, pH adjuster, buffers, neutralizers, colorants, and water-soluble biologically active compounds such as glycerin, glycols, extracts, and the like. The concentration of the water phase, as a weight percent of the total composition, may range from about 10% to about 90%, such as from about 20% to about 80%, such as from about 40% to about 60%. In certain embodiments, the concentration of the water phase may be greater than the concentration of the oil phase.

Compositions of the present disclosure may include one or more organic UV filters. Organic UV filters that are useful in the present disclosure may be cosmetically-acceptable compounds that absorb radiation in the solar UV range (290-400 nm) and may generally be soluble in one or more organic solvents and/or water.

Examples of organic UV filters include without limitation p-aminobenzoic acid and salts and derivatives thereof; anthranilate and derivatives thereof; salicylate and derivatives thereof; cinnamic acid and derivatives thereof; dihydroxycinnamic acid and derivatives thereof; camphor and salts and derivatives thereof; trihydroxycinnamic acid and derivatives thereof; dibenzalacetone naptholsulfonate and salts and derivatives thereof; benzalacetophenone naphtholsulfonate and salts and derives thereof; dihydroxy-naphthoic acid and salts thereof; o-hydroxydiphenyldisulfonate and salts and derivatives thereof; p-hydroxdydiphenyldisulfonate and salts and derivatives thereof; coumarin and derivatives thereof; diazole derivatives; quinine derivatives and salts thereof; quinoline derivatives; hydroxyl-substituted benzophenone derivatives; naphthalate derivatives; methoxy-substituted benzophenone derivatives; uric acid derivatives; vilouric acid derivatives; tannic acid and derivatives thereof; hydroquinone; benzophenone derivatives; 1, 3, 5- triazine derivatives; phenyldibenzimidazole tetrasulfonate and salts and derivatives thereof; terephthalyidene dicamphor sulfonic acid and salts and derivatives thereof; methylene bis-benzotriazolyl tetramethylbutylphenol and salts and derivatives thereof; bis-ethylhexyloxyphenol methoxyphenyl triazine and salts, diethylamino hydroxyl benzoyl and derivatives thereof; and combinations of the foregoing.

In some embodiments, personal care product (e.g. sunscreen) compositions may include one or more UV filters selected from the group comprising Octocrylene, salicylic acid esters (e.g., Ethylhexyl salicylate and Homomenthyl salicylate, also known as Homosalate), p-methoxycinnamic acid esters (e.g., Ethylhexyl methoxycinnamate and Isoamyl methoxycinnamate), Avobenzone (also known as Butyl methoxydibenzoylmethane), Oxybenzone (also known as Benzophenone-3), Bemotrizinol (also known as Bis-ethylhexyloxyphenol methoxyphenyl triazone), Bisoctrizole (also known as Methylene bis-benzotriazolyl Tetramethylbutylphenol), 4-Methylbenzylidene camphor, Diethylamino hydroxyl benzoyl hexyl benzoate, Drometrizole trisiloxane, Ethylhexyl triazone, Diethylhexyl butamido triazone, Ecamsule (also known as terephthalyidene dicamphor sulfonic acid and salts thereof) and Menthyl anthranilate.

According to some embodiments (e.g., in compositions intended for sale in the United States), UV filters may include Octocrylene, Avobenzone, Homosalate, Octisalate (Ethylhexyl salicylate), Octinoxate (Ethylhexyl methoxycinnamate), and Oxybenzone.

In some embodiments of the present disclosure, compositions of the oil phase may comprise solvents and emollients selected from the group comprising fatty acid esters, fatty alcohol esters, esters of Guerbet alcohols, benzoic acid esters, esters of dibasic acids, esters of polyprotic acids, esters of polyhydric alcohols including triglycerides, Polyethylene glycols and esters thereof, Polypropylene glycols and esters thereof, polyesters, silicones, organically-modified silicones, hydrocarbon compounds comprising, mineral oils including paraffinic oils, naphthenic oils, and aromatic oils and polymers derived from hydrocarbon compounds such as hydrogenated polyisobutene and polypropylene.

In certain embodiments, the solvents and emollients in the oil phase may be C12-15 alkyl benzoate available as FINSOLV® TN from Innospec, Salisbury, North Carolina, Butyloctyl salicylate available as HALLBRITE® BHB from HallStar, Chicago, Illinois, and Ethylhexyl olivate available as Sensolene™ from HallStar Italia, Arcore, Italy.

An oil phase may comprise, according to some embodiments, compounds that stabilize other compounds present in the oil phase by, for example, preventing their degradation due to exposure to solar radiation or to oxidation from exposure to the atmosphere. In certain embodiments, the oil phase may contain stabilizers selected from the group comprising Ethylhexyl methoxycrylene (e.g. SOLASTAY® S1 from HallStar, Chicago, Illinois), Diethylhexyl syringylidene malonate (e.g. OXYNEX™ ST from EMD Millipore, Philadelphia, Pennsylvania), Diethylhexyl 2,6-naphthalate (e.g., CORAPAN™ TQ from Symrise, Holzminden, Germany), Polyester-8 (e.g. POLYCRYLENE® from HallStar, Chicago, Illinois), Polyester-25 (e.g. HallStar, Chicago, Illinois), Undecylcrylene dimethicone (e.g. HALLBRITE® PSF from HallStar, Chicago, Illinois), Pentaerythrityl Tetra-di-t-butyl Hydroxyhydrocinnamate (e.g. Tinogard TT from BASF, Ludwigshafen, Germany), Octadecyl Di-t-butyl-4-hydroxyhydrocinnamate (e.g. Tinogard TS from BASF, Ludwigshafen, Germany), Benzotriazolyl dodecyl p-cresol (e.g. Tinogard TL from BASF, Ludwigshafen, Germany), Tris (e.g. Tetramethylhydroxypiperidinol), Citrate (e.g. Tinogard QS from BASF, Ludwigshafen, Germany), Sodium Benzotriazolyl Butylphenol Sulfonate (e.g. Tinogard HS from BASF, Ludwigshafen, Germany), Bumetrizole (e.g. Tinogard AS from BASF, Ludwigshafen, Germany), Butylated hydroxyltoluene (BHT), Butylated hydroxyanisole (BHA), Tocopherol, and many others. Other stabilizers known to confer stability against photo- and oxidative degradation in personal care product compositions may also be suitable substitutes.

Some embodiments of the present disclosure may advantageously comprise a film-forming polymer in the oil phase. A film-forming polymer may confer on the composition some amount of water- and wear-resistance after it is applied to the skin. Suitable film forming polymers may include natural polymers such as polysaccharides or proteins and synthetic polymers such as polyesters, polyacrylics, polyurethanes, vinyl polymers, polysulfonates, polyureas, polyoxazolines, and the like. Specific examples of film-forming polymers may include, for example, acrylic homopolymers or copolymers with hydrophobic groups such as acrylates/octylacrylamide copolymers including DERMACRYL 79 available from Akzo Nobel, Bridgewater, New Jersey; dimethicone/acrylates dimethicone copolymer available as X-22-8247D from Shin-Etsu of Japan; hydrogenated dimer dilinoleyl/dimethylcarbonate copolymer, available as COSMEDIA DC from Cognis Corporation of Ambler, Pennsylvania; copolymers of vinylpyrrolidone and a long-chain alpha-olefin, such as those commercially available as GANEX V-220 and GANEX V-216 and vinylpyrrolidone/tricontanyl copolymer commercially available as GANEX WP660 from Ashland Chemicals of Covington, Kentucky. VA/butyl maleate/isobornyl acrylate copolymer available as ADVANTAGE® PLUS from Ashland, Covington, Kentucky. According to some embodiments, Acrylates/Dimethicone copolymer (SILDERM ACRYLATES from Active Concepts) may advantageously confer good water resistance when present at a concentration of about 3% by weight in a composition tested as prescribed by the U.S. Food and Drug Administration to determine the Sun Protection Factor (SPF) after immersion in water for 80 minutes. Such a composition may exhibit very little whitening when applied to wet skin. Other film forming polymers known to confer water resistance to personal care product compositions may also be suitable substitutes.

The oil phase of the present disclosure may also contain an emulsifying compound that may aid in the dispersion of the oil phase into the water phase. An emulsifying compound may be a liquid at room temperature and have a hydrophilic-lipophilic balance (HLB) less than about 5. In certain embodiments of the present disclosure, an emulsifying compound may be Glyceryl Oleate (e.g. HALLSTAR GMO from HallStar, Chicago, Illinois), which may have an HLB of 3.8 and may be a liquid at room temperature. In some embodiments, Glyceryl Oleate may be present at a concentration by weight of 0-1%, such as 0.1-0.8%. In some embodiments, Glyceryl Oleate may be present at a concentration by weight of 0.3-0.5%. Certain other embodiments of the present disclosure may contain Sorbitan Oleate (e.g. SPAN 80 from Croda, Edison, New Jersey), which may have an HLB of 4.3 and may be a liquid at room temperature. In some embodiments, Sorbitan Oleate may be present at a concentration by weight of 0-1%, such as 0.1-0.8%. In some embodiments, Sorbitan Oleate may be present at a concentration of 0.2-0.4%.

The water phase of compositions of the present disclosure may contain, in addition to water, glycols or polyhydric alcohols such as glycerin, propylene glycol, butylene glycol, or hexalene glycol known to exert a moisturizing humectant effect on the skin. Glycols or polyhydric alcohols may be present at concentrations of 0-10% of the composition, such as 1%-7%, such as 3%-5%. In addition, the water phase of the present disclosure may comprise any of various other cosmetically acceptable ingredients such as preservatives, fragrances, dyes, antioxidants, skin-benefit agents, extracts, neutralizing agents, and the like.

Certain copolymers of acrylic acid, notably Acrylate/C10-30 alkyl acrylates crosspolymers, commonly known as carbomers (e.g. PEMULEN TR-1 and PEMULEN TR-2 from Lubrizol) may be effective as primary emulsifiers. Such primary emulsifiers may have a small lipophilic portion in addition to a large hydrophilic portion.

Whereas water soluble polymers (e.g. Carbopol®) may have usefulness as secondary oil-in-water (o/w) emulsion stabilizers, carbomers (e.g. Pemulen polymers) may actually form o/w (oil-in-water) emulsions. The lipophilic portion may adsorb at the oil-water interface, and the hydrophilic portion may swell in the water and thereby form a gel network around oil droplets to provide emulsion stability to a broad range of oils. For the purposes of formulating a personal care product (e.g. sunscreen) applies clear (e.g., becomes transparent when applied) to wet skin and remains clear after applicationsome carbomers (e.g., Pemulen® polymeric emulsifiers) may not be suitable in some circumstances because they may exhibit strong whitening upon application to wet skin.

In an unexpected result, applicants have found that (a) certain Carbomer Copolymers and (b) polymers of acrylic acid that are either crosslinked with allyl sucrose or allyl pentaerythritol, hereinafter referred to generically as Carbomer Homopolymers," or (c) polymers that contain a block copolymer of polyethylene glycol and a long chain alkyl acid ester, hereinafter referred to as "Carbomer Interpolymers," that may have been previously thought of as useful only as secondary oil-water emultion stabilizers, when substituted for Pemulen polymeric emulsifiers, may not only create stable emulsions but may also help achieve personal care product (e.g. sunscreen) formulations that apply clear to wet skin and remain clear after application while retaining their other functions (e.g. UV protecting and water resistance properties).

Carbomer Copolymers, Carbomer Homopolymers and Carbomer Interpolymers that are suitable for the purpose of formulating topical personal care products, including sunscreens, that quickly clarify on wet skin and remain clear may include the Carbomer Homopolymers: Carbopol® 71G NF polymer, Carbopol® 971P, and Carbopol 981 and 981 NF polymer, Carbopol® 974P NF polymer, Carbopol® 5984 EP polymer, and Carbopol® 980 and 980 NF polymer, Carbopol® ETD 2050 polymer, and Carbopol® Ultrez 30 polymer, and chemical equivalents thereof. They may also include the Carbomer Interpolymers: Carbopol® Ultrez 10 and Ultrez 10 NF polymer and Carbopol® ETD 2020, and chemical equivalents thereof. They may also include Carbomer Copolymers: Carbopol® Ultrez 20 polymer and Carbopol 21 polymer, and chemical equivalents thereof. Other appropriate compounds may include Carbopol® Ultrez 20 Polymer, Carbopol® Ultrez 21 Polymer, Carbopol® Ultrez 30 Polymer, and chemical equivalents thereof.

Carbopol® ETD 2050 is an exemplary Carbomer Homopolymer that may be commercially available from Lubrizol that may be suitable for the purpose of formulating topical personal care products (e.g. sunscreens) that applies clear on wet skin and remain clear.

Specific Carbomer Homopolymers commercially available from Lubrizol that may be suitable for the purpose of formulating topical personal care products (e.g. sunscreens) that apply clear on wet skin and remain clear may include Carbopol® 71G NF polymer, Carbopol® 971P, and Carbopol 981 and 981 NF polymer, Carbopol® 974P NF polymer, Carbopol® 5984 EP polymer, and Carbopol® 980 and 980 NF polymer, Carbopol® ETD 2050 polymer, and Carbopol Ultrez 30 polymer, and chemical equivalents thereof.

Specific Carbomer Interpolymers commercially available from Lubrizol that may be suitable for the purpose of formulating topical personal care products (e.g. sunscreens) that apply clear on wet skin and remain clear may include Carbopol® Ultrez 10 and Ultrez 10 NF polymer and Carbopol® ETD 2020, and chemical equivalents thereof.

The foregoing list is not intended as comprehensive and manufacturers other than Lubrizol may offer chemically similar or identical grades of the foregoing Carbomer Copolymers, Carbomer Homopolymers and Carbomer Interpolymers. Other suitable Carbomer Copolymers, Carbomer Homopolymers and Carbomer Interpolymers may be available from Ashland Inc., Covington, Kentucky may include Ashland Carbomer 940, Ashland Carbomer 980, Ashland Carbomer 941, and Ashland Carbomer 981. Carbomers from other suppliers may also be suitable for the purpose of formulating topical personal care products (e.g. sunscreens) that apply clear to wet skin and remain clear.

Certain embodiments of the present disclosure may contain Carbomer Homopolymers (e.g. Carbopol ETD 2050) in concentrations of about 0.1% to about 0.5%, such as about 0.15% to about 0.4%, such as about 0.3% to about 0.4% by weight of the compositions.

Certain embodiments of the present disclosure may contain Carbomer Interpolymers in concentrations of about 0.1% to about 0.5% by weight of the composition, such as about 0.1% to about 0.4%, such as about 0.15% to about 0.3%.

Certain embodiments of the present disclosure may contain a Carbomer Copolymers in concentrations of about 0.1% to about 0.5% by weight of the composition such as about 0.15% to about 0.25%.

According to some embodiments of the present disclosure, it may be desirable for the composition to be applied to wet skin such that there may be little or no whitening; i.e. no more than a momentary discoloration or masking of the skin's natural color and appearance while the skin is still wet. Applicants have found that formulating compositions of the present disclosure with Carbomer Homopolymers in concentrations of about 0.1% to about 0.5%, such as about 0.15% to about 0.4%, such as about 0.3% by weight of the compositions may provide the compositions with the ability to clarify almost instantly on wet skin as soon as spreading begins.

According to some embodiments of the present disclosure, it may be desirable for compositions to maintain their integrity (stability) when subjected to high or low temperatures for extended periods of time. Applicants have found that formulating compositions of the present disclosure with Carbomer Copolymers or Carbomer Interpolymers in concentrations of about 0.1% to about 0.5%, such as about 0.15% to about 0.4%, such as about 0.3% by weight of the composition may be sufficient to stabilize compositions even when continually subjected to temperatures of about 60o C or higher or about 5o C or lower for at least two weeks, even in the absence of any emulsifying compounds. However, compositions may optionally comprise low levels of low HLB emulsifying compounds such as Glyceryl oleate or Sorbitan oleate with HLB values of 3.8 and 4.3 respectively in concentrations of about 0% to about 1%, such as about 0.1% to about 0.6%, such as about 0.2% to about 0.4%, for use primarily as a dispersant. Applicants have also found that formulating compositions of the present disclosure with Carbomer Copolymers or Carbomer Interpolymers in concentrations of about 0.25% to about 0.5%, such as about 0.3%, may provide the composition with the ability to clarify almost instantly on wet skin as soon as spreading begins. In certain embodiments, for example when the Carbomer Copolymer is Carbopol® Ultrez 20, the applicants have found that formulating compositions of the present disclosure with a Carbomer Copolymer in a concentration of about 0.1% to about 0.5%, such as about 0.3% may provide compositions with the ability to maintain their integrity (stability) when subjected to high or low temperatures for extended periods of time.

Consequently, compositions of the present disclosure may include a Carbomer Copolymer or a Carbomer Interpolymer in combination with a Carbomer Homopolymer in a combined concentration of about 0.3% to about 0.5%. The Carbomer Copolymer or Carbomer Interpolymer may contribute stability to the composition and the Carbomer Homopolymer may contribute almost instant clarity when applied and spread onto wet skin. Certain embodiments of the present disclosure may contain 0% Carbomer Homopolymer and 0.3% Carbomer Interpolymer and may exhibit both excellent clarity on wet skin and excellent stability. Certain embodiments of the present disclosure may contain about 0.15% Carbomer Homopolymer and about 0.15% Carbomer Interpolymer or Carbomer Copolymer in combination and may exhibit both excellent clarity on wet skin and stability.

In some embodiments, it may be desirable to produce a stable composition comprising an oil phase that may be greater than 30% by weight of the composition. The greater the oil phase as a percent of the total composition, the more difficult it may be to formulate a stable composition (i.e. one that may not exhibit signs of instability after prolonged exposure to both high and low temperatures). Applicants have found that certain embodiments of the present disclosure containing oil phases as great as about 41.4% by weight of the composition may be both emulsified and stabilized by a non-emulsifying grade of Carbomer Copolymer alone (See Examples 10 and 16 in Table 3), and by Carbomer Interpolymer alone (See Examples 2, 4, 5 6, 7, 8, 13, and 15 in Table 3), and by a non-emulsifying grade of Carbomer Homopolymer alone (see Examples 12 and 17 in Table 3), and by non-emulsifying grades of Carbomer Interpolymer and Carbomer Homopolymer in combined amounts of as little as about 0.3% by weight of the composition (See Examples 2 and 8 in Table 3). The foregoing examples and the examples presented below are merely illustrative and not intended as a comprehensive survey of all possible stable, non-whitening compositions that may be produced by using non-emulsifying grades of Carbomer Copolymer, Carbomer Interpolymer, and Carbomer Homopolymer alone or in combination according to embodiments of the present disclosure.

In some embodiments, Carbopol® Polymers may be dispersed as aqueous solutions. Single particles of Carbopol® may wet out very rapidly when exposed to water. Carbopol® polymers may form clumps of particles when haphazardly disperse in polar solvents. Dispersion of Carbopol® polymers may be achieved by taking advantage of its small particle size. Advantages related to its small particle size may be lost if Carbopol® polymers are added too fast. Accordingly, in some dispersions, Carbopol® polymers should be slowly and carefully added into the dispersion medium while the mix is being stirred rapidly. Agitation may enhance the rate of Carbopol® solvation. Various techniques to promote Carbopol® dispersion such as indirect addition and direct addition may be employed.

In some embodiments of the present disclosure, compositions may further comprise pharmaceuticals or pharmacologically active ingredients. Compositions may be formulated as a pharmaceutical dosage form. Compositions may be formulated as a drug delivery system. A pharmaceutical may comprise pharmacologically active chemical entity that may be transdermally absorbed. Examples of pharmacologically active chemical entity that may be transdermally absorbed may include acne agents, anesthetics, anti-infective, antibiotics, antifungals, antihistamines, antipsoriatics, antivirals, astringents, debriding agents, depigmenting agents, emollients, keratolytics, steroids, and/or non-steroidal anti-inflammatories. Other pharmacologically active chemical entities or topical dermatological agents may be appropriate depending on the desired composition of an embodiment of the present disclosure.

As will be understood by those skilled in the art who have the benefit of the instant disclosure, other equivalent or alternative compositions, devices, methods, and systems for formulating a personal care product that applies clear to wet skin and remains clear can be envisioned without departing from the description contained herein. Accordingly, the manner of carrying out the disclosure as shown and described is to be construed as illustrative only.

Each disclosed method and method step may be performed in association with any other disclosed method or method step and in any order according to some embodiments. Where the verb "may" appears, it is intended to convey an optional and/or permissive condition, but its use is not intended to suggest any lack of operability unless otherwise indicated. Persons skilled in the art may make various changes in methods of preparing and using a composition, device, and/or system of the disclosure. For example, a composition, device, and/or system may be prepared and or used as appropriate for animal and/or human use (e.g., with regard to sanitary, infectivity, safety, toxicity, biometric, and other considerations). Elements, compositions, devices, systems, methods, and method steps not recited may be included or excluded as desired or required.

Also, where ranges have been provided, the disclosed endpoints may be treated as exact and/or approximations as desired or demanded by the particular embodiment. Where the endpoints are approximate, the degree of flexibility may vary in proportion to the order of magnitude of the range. For example, on one hand, a range endpoint of about 50 in the context of a range of about 5 to about 50 may include 50.5, but not 52.5 or 55 and, on the other hand, a range endpoint of about 50 in the context of a range of about 0.5 to about 50 may include 55, but not 60 or 75. In addition, it may be desirable, in some embodiments, to mix and match range endpoints. Also, in some embodiments, each figure disclosed (e.g., in one or more of the examples, tables, and/or drawings) may form the basis of a range (e.g., depicted value +/- about 10%, depicted value +/- about 50%, depicted value +/- about 100%) and/or a range endpoint. With respect to the former, a value of 50 depicted in an example, table, and/or drawing may form the basis of a range of, for example, about 45 to about 55, about 25 to about 100, and/or about 0 to about 100. Disclosed percentages are weight percentages except where indicated otherwise.

These equivalents and alternatives along with obvious changes and modifications are intended to be included within the scope of the present disclosure. Accordingly, the foregoing disclosure is intended to be illustrative, but not limiting, of the scope of the disclosure as illustrated by the appended claims.

### EXAMPLES

Specific example embodiments of the disclosure may be illustrated by one or more of the examples provided herein.

### EXAMPLE 1: Compositions and Method of Preparation

The following composition was prepared according to the ingredient list in Table 1 and the preparative procedure detailed below.

**Table 1: Example Product Composition Formula JZ5-280B**

| Item | Ingredient INCI Name (Trade Name [Drug Name], Supplier) | % Wt |
|---|---|---|
| 1 | Homosalate [Homosalate] | 10.00 |
| 2 | Octocrylene [Octocrylene] (Neo Heliopan ® 303, Symrise) | 6.00 |
| 3 | Phenethyl Benzoate (X-Tend™ 226, Ashland / ISP) | 5.00 |
| 4* | Ethylhexyl Methoxycrylene (SolaStay ® S₁, HallStar) | 4.00 |
| 5* | Butyloctyl Salicylate (HallBrite ® BHB, HallStar) | 3.00 |
| 6 | Butyl Methoxydibenzoylmethane [Avobenzone] (Neo Heliopan ® 357, Symrise) | 3.00 |
| 7 | Benzophenone-3 [Oxybenzone] (Neo Heliopan ® BB, Symrise) | 6.00 |
| 8* | Glyceryl Oleate (HallStar ® GMO, HallStar) | 0.40 |
| 9 | Acrylates/Dimethicone Copolymer (SilDerm Acrylate, Active Concepts) | 3.00 |
| 10 | Styrene/Acrylates Copolymer (Sunspheres ™ Powder, Dow Chemical) | 2.00 |
| 11 | Water (*aqua*) (deionized) | 49.96 |
| 12 | Disodium EDTA (Versene ™ NA, Dow Chemical) | 0.05 |
| 13 | Carbomer (Carbopol ® Ultrez ™ 10, Lubrizol / Noveon) | 0.30 |
| 14 | Phenoxyethanol, Caprylyl Glycol, Chlorphenesin (Mikrokill ® COS, Arch Chemicals) | 1.00 |
| 15 | Butylene Glycol (1,3-Butylene Glycol, Celanese) | 3.00 |
| 16 | Triethanolamine (Triethanolamine 99%, Dow Chemical) | 0.29 |
| 17* | Bis-(Benzyl undecanoate) dimethicone (Proposed) (Trade Name TBD, HallStar) | 1.00 |
| 18 | Dimethicone (Dow Corning ® Q7-9120 Silicone Fluid. 20CST. Dow Corning) | 2.00 |

| | | |
|---|---|---|
| * = available from HallStar | | |

### PREPARATIVE PROCEDURE

To the main vessel add 1 - 5, start mixing, add 6 and 7, heat to 55°C. When 6 and 7 are totally dissolved, add 8 and mix well, add 9 and mix well. When 1 - 9 is homogeneous, add 10 and mix until 10 is well dispersed.

Add 11 to a second vessel, dissolve 12 with continued mixing and then add 13, stirring until fully incorporated. Add premix of 14 and 15 into 11 - 13 and mix well.

Start homogenizing the oil phase 1 - 10 with cooling. Slowly add the water phase (11 - 15) into the oil phase 1 - 10. Add 16 with prop homogenizing. Add 17 and 18 continue homogenizing until fully incorporated.

When completely smooth, perform final quality assurance checks.

### PRODUCT PROPERTIES (25°C)

Appearance: light yellow cream
Viscosity (RV, T-D, 5, 20 & 100 rpm, cP): 54000, 17200 & 5360
pH: 5.5

Example 1 was determined to have a 80 minute Water Resistant SPF of 66 by Florida Sunscreen Testing Inc., a clinical testing laboratory, according to SOP #2011-01 80 Minute Water Resistant SPF Testing, which is the procedure set forth by the U.S. Food and Drug Administration in 21 CFR Sec. 201.327, subpart (i), SPF Test Procedure, Sunscreen Drug Products for Over-the-Counter Human Use, Final Monograph, Federal Register, Vol. 76, No. 117, June 17, 2011. Specifically, sunscreen products may contain the statement "water resistant" if a sunscreen product was shown to retain the labeled SPF value after 40 minutes of water immersion. Further, a product may contain the statement "very water resistant" if a sunscreen product was shown to retain the labeled SPF value after 80 minutes of water immersion. The PFA values in Examples 1-14 and the SPF values displayed for Examples 2-14 were determined according to the in vitro testing methods detailed in HallStar Technical Publication "In vitro SPF Analysis,".

### In vitro SPF Analysis

### A. Equipment

1. Instrument - UV2000S UV Transmittance Analyzer (Labsphere, Inc.)
2. Software - UV 2000 Version 1.1.0.0 (Labsphere, Inc)
3. Analytical balance - d= 0.1 mg

### B. Materials and Conditions

1. Substrate - PMMA: Helioplate Tid HD 6 plates (HelioScreen Labs)
2. Software settings:
   a. Integral Limits UV-B- 290-320nm; UV-A -- 320-400nm; SPF -- 290-400nm
   b. Spectral Irradiance Noon, July 3, Albuquerque. NM
   c. SPF Spectral Irradiance and Erythemal Effectiveness settings as set by manufacturer

### C. Methods

1. Sample plate preparation for blank: Tare the analytical balance to zero and place sample plate on the balance. Apply 3 - 4 mg petrolatum to the plate. Take the plate from the balance and, using one finger with a powder-free finger cot on it, spread the petrolatum until the plate becomes transparent.
2. Sample plate preparation for test Tare the analytical balance to zero and place sample plate on the balance. Record the plate weight as "A " Add 32.5 mg test substance to the plate so the balance now indicates a mass of ("A" + 32.5) mg. Take the plate from the balance and immediately spread the test substance with several rapid back and forth motions using one finger with a powder -free finger cot on it. Repeat several times, rotating the plate 90 degrees each time, until a very thin, uniform layer of test substance results. Then place the plate in a dark place and let stand for 20 minutes.
3. SPF Test -- The blank plate from (C 1) above is placed on the UV Transmittance Analyzer. Open the software on Study and then Method. Select Colipa Guideline (2007). Follow the instruction to scan the blank plate. When the sample plate(s) from (C 2) above is (are) ready, again follow the instructions for the pre-irradiation location scans of the sample plate(s). For each sample, enter in vivo or expected SPF number in the space for Labelled SPF. SPF Mean and UVAPF, Pre-irradiation, are shown in the result table. Irradiate the sample plate and then run the post-irradiation location scans per instructions. A report is generated with UVAPF Mean, Ratio (SPF in vivo AJVAPF), and UVA Balance.

The Example 1 composition applied clear to wet skin and represents a significant improvement over the aforementioned compositions of Singleton. Example 1 was stable when subjected to both 60oC for one month and to 5oC for one month.

### EXAMPLE 2: Compositions and Method of Preparation

Example 2, summarized in **Table 3** below, was prepared from the same ingredient list and using the same preparative procedure as Example 1 except as noted in Table 1 (0.3% Ultrez 10 was replaced by 0.15% Ultrez 10 and 0.15% Carbopol 980) and ingredient #10 was reduced from 2% to 1% by weight of the composition. Example 2 exhibits excellent clarity when applied to wet skin and stability when subjected to both 60oC for one month and to 5oC for one month.

### EXAMPLE 3: Composition and Method of Preparation

The following composition was prepared according to the ingredient list in Table 2 and the preparative procedure detailed below.

**Table 2: Example Product Composition FORMULA JZ6-71**

| Item | Ingredient INCI Name (Trade Name [Drug Name], Supplier) | % Wt |
|---|---|---|
| 1 | Homosalate [Homosalate] | 10.00 |
| 2 | Octocrylene [Octocrylene] (Neo Heliopan ® 303, Symrise) | 7.00 |
| 3 | Ethylhexyl Salicylate [Octisalate] | 5.00 |
| 4* | Ethylhexyl Methoxycrylene (SolaStay ® S₁, HallStar) | 2.00 |
| 5* | Polyester-25 (Trade name TBD, HallStar) | 2.00 |
| 6* | Butyloctyl Salicylate (HallBrite ® BHB, HallStar) | 3.00 |
| 7* | Glyceryl Oleate (HallStar ® GMO, HallStar) | 0.40 |
| 8* | Ethylhexyl Olivate (Sensolene ®, HallStar Italia) | 2.00 |
| 9 | Acrylates/Dimethicone Copolymer (SilDerm Acrylate, Active Concepts) | 2.00 |
| 10 | Butyl Methoxydibenzoylmethane [Avobenzone] (Neo Heliopan ® 357, Symrise) | 3.00 |
| 11 | Benzophenone-3 [Oxybenzone] (Neo Heliopan ® BB, Symrise) | 6.00 |
| 12 | Styrene/Acrylates Copolymer (Sunspheres ™ Powder, Dow Chemical) | 1.00 |
| 13 | Water (*aqua*) (deionized) | 52.00 |
| 14 | Disodium EDTA (Versene ™ NA, Dow Chemical) | 0.05 |
| 15 | Carbomer (Carbopol ® Ultrez ™ 10, Lubrizol / Noveon) | 0.30 |
| 16 | Phenoxyethanol, Caprylyl Glycol, Chlorphenesin (Mikrokill ® COS, Arch Chemicals) | 1.00 |
| 17 | Butylene Glycol (1,3-Butylene Glycol, Celanese) | 3.00 |
| 18 | Triethanolamine (Triethanolamine 99%, Dow Chemical) | 0.25 |

| | | |
|---|---|---|
| * = available from HallStar | | |

### PREPARATIVE PROCEDURE

In a separate vessel combine 1 - 9 and start heating with mixing to 50°C. Add 10 and 11 and mix until clear. Add 12 and mix until 12 is well dispersed.

Add 13 to main vessel and start center-stir (prop) mixing. Disperse 14 in 13 and then add 15 with continuing mixing this water phase until fully dispersed. Add premix of 16 and 17, add mix until homogeneous. Switch to sweep mixing right before combining two phases.

Add the oil phase to the water phase under sweep mixing. Add 18 with strong sweep mixing.

When the emulsion is fully formed (white and thickened), replace any water lost during processing. Keep mixing until batch is again homogeneous. When completely smooth, perform final quality assurance checks.

### PRODUCT PROPERTIES (25°C)

Appearance: light yellow cream
Viscosity (RV, T-D, 5, 20 & 100 rpm, cP): 42400, 14300, 4600
pH: 5.5

Example 3 was tested by in vitro testing methods detailed in the aforementioned HallStar Technical Publication "In vitro SPF Analysis" and found to achieve SPF 62 and a UVA-PF (UVA Protection Factor) of 24, and to have a Critical Wavelength of 376 nm. The composition exhibits excellent clarity when applied to wet skin and stability when subjected to both 60oC for one month and to 5oC for one month. The preparative procedure used in Example 3 was also employed to prepare Examples 4-14 listed in **Table 1** below. Examples 3-14 in **Table 1** share the same ingredient list as Example 2 except for noted substitutions in ingredients Nos. 7, and 15, and substitutions not noted, in ingredient No. 8, which is included at 2% by weight of the compositions for the purpose of improving aesthetic (i.e. skin feel), has no noticeable effect on either stability or whitening, and is therefore not pertinent to the present disclosure.

**Table 3: Examples of Compositions of the Present Disclosure Made and Tested**

| **Example #** | **Notebook Number** | **Carbomer(s)** | **Low HLB Emulsifier** | **Stability at 5°C** | **Stability at 60°C** | **Whitening or Not** | **SPF/PFA** |
|---|---|---|---|---|---|---|---|
| 1 | JZ5-280B | 0.3% Ultrez 10 (CI) | 0.4% GMO | Pass 4 months* | Pass 4 months* | Slight | 66/26 |
| 2 | JZ6-12B | 0.15% Ultrez 10 (CI)/0.15% 980NF (CH) | 0.4% GMO | Pass 3 months* | Pass 3 months* | Not | 60/24 |
| 3 | JZ6-71 | 0.3% Ultrez 10 (CI) | 0.4% GMO | Pass 1 month* | Pass 1 month* | Not | 62/24 |
| 4 | JZ6-83 | 0.3% Ultrez 10 (CI) | 0% GMO | Pass - 1 month | Pass - 1 month | Not | 65/23 |
| 5 | JZ6-84 | 0.3% Ultrez 10 (CI) | 1.0% GMO | Pass* - 1 month | Pass* - 1 month | Not | 65/24 |
| 6 | JZ6-85 | 0.3% ETD 2050 (CH) | 0.4% GMO | Pass - 1 month | Pass - 1 month | Not | 60/22 |
| 7 | JZ6-114 | 0.3% Ultrez 20 (CC) | 0.4% GMO | Pass - 1 month | Pass - 1 month | Whitening | 60/22 |
| 8 | JZ-113 | 0.15% Ultrez 20 (CC)/0.15% 980 (CH) | 0.4% GMO | Pass - 1 month | Pass - 1 month | Not | 60/20 |
| 9 | JZ6-87 | 0.3% ETD 2050 (CH) | 0.4% GMO | Pass - 1 month | Pass - 1 month | Not | 60/20 |
| 10 | JZ6-91 | 0.3% Ultrez 10 (CI) | 0.2% SMO | Pass - 1 month | Pass - 1 month | Not | 62/20 |
| 11 | JZ6-92 | 0.3% Ultrez 10 (CI) | 0.4% SMO | Pass - 1 month | Pass - 1 month | Not | 65/20 |
| 12 | JZ6-98 | 0.3% ETD 2020 (CI) | 0.4% GMO | Pass - 1 month | Pass - 1 month | Not | 60/20 |
| 13 | JZ6-99 | 0.3% Ultrez 21 (CC) | 0.4% GMO | Pass - 1 month | Pass - 1 month | Not | 62/20 |
| 14 | JZ6-100 | 0.3% Ultrez 30 (CH) | 0.4% GMO | Pass - 1 month | Pass - 1 month | Not | 60/20 |

| | | | | | | | |
|---|---|---|---|---|---|---|---|
| GMO = Glyceryl monooleate; SMO = Sorbitan monooleate; CC = Carbomer Copolymer; CH = Carbomer Homopolymer; CI = Carbomer Interpolymer; Pass = The physical appearance of the sample remains unchanged. | | | | | | | |

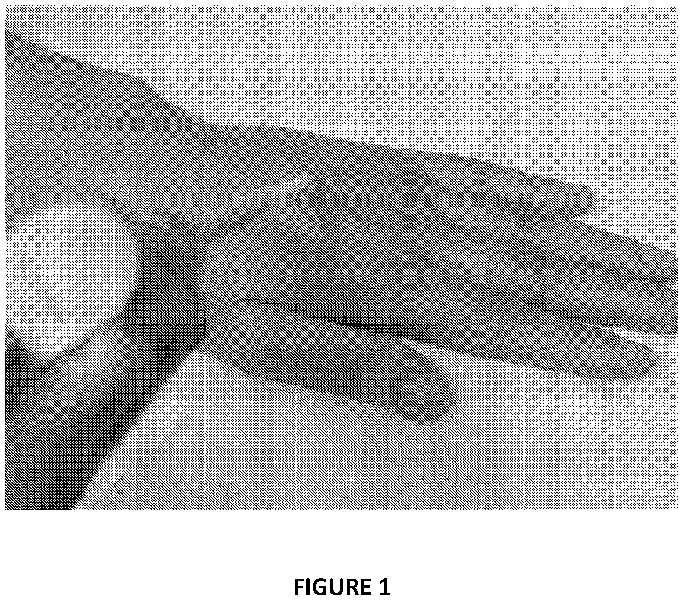

Applications of specific example embodiments of the present disclosure may be further understood by reference to the accompanying Figures 1-12. Figures 1-12 may be representative of results obtained according to various embodiments of the present disclosure.

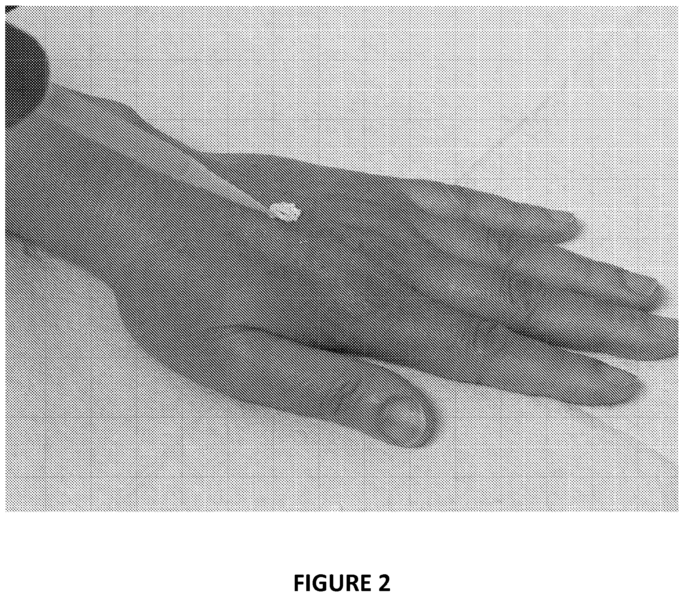

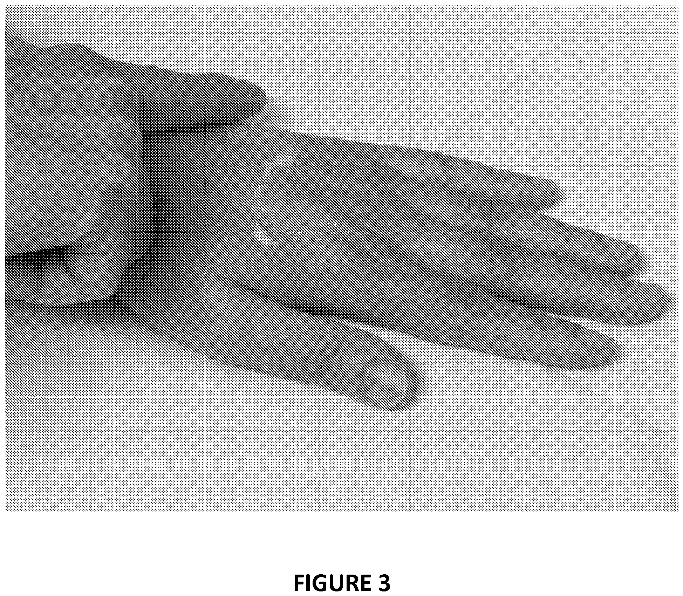

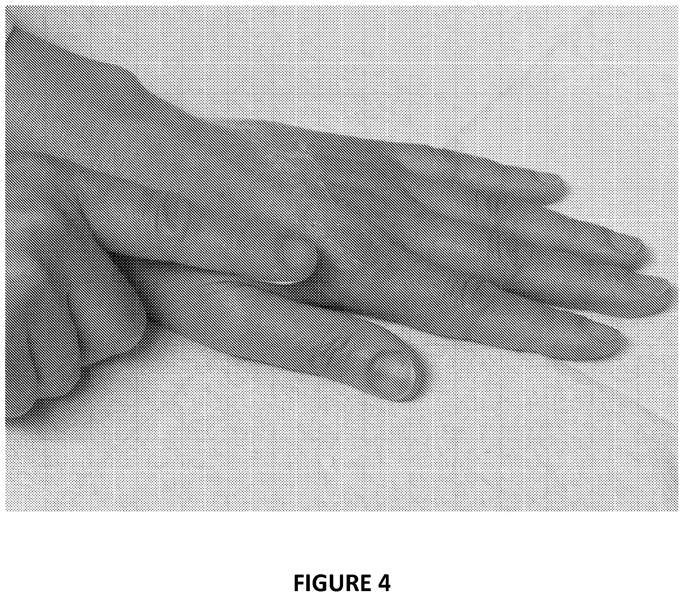

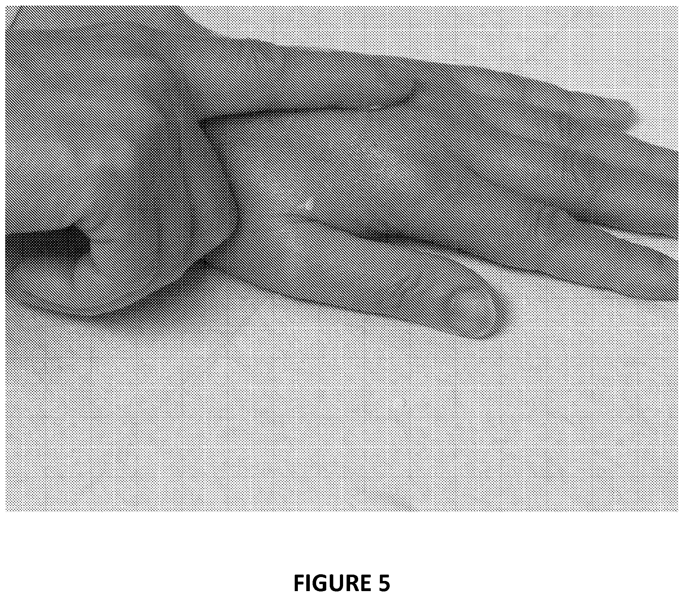

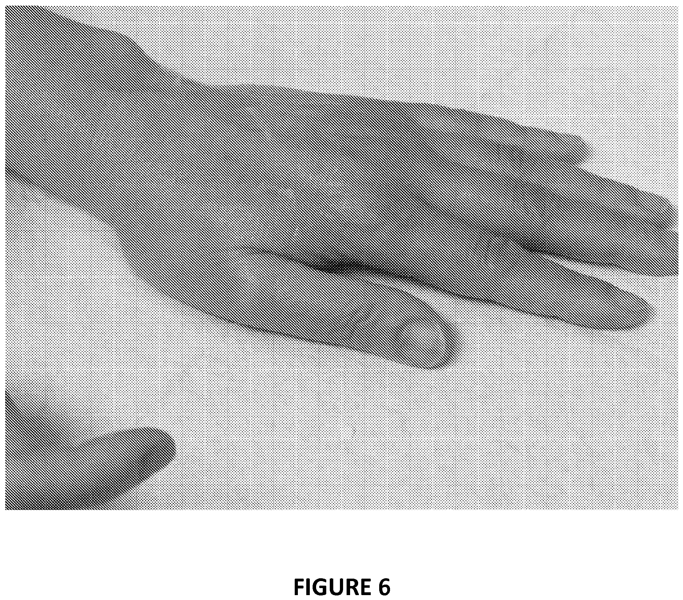

Figures 1-6 illustrate the application of a personal care product composition according to a specific example embodiment of the present disclosure. In Figure 1, 50 µl of deionized water is applied to the surface of a hand. As seen in Figure 1, the water remains on the skin surface and provides moisture and wetness. In Figure 2, 50 mg of a personal care product composition according to a specific example embodiment of the present disclosure is applied to the same region where the water was applied. The swiping motion of a finger may promote mixing of the water and personal care product composition. Other motions such as a rubbing motion or circular wiping motions may be appropriate to achieving similar results. In some circumstances, it may be advantageous to provide a personal care product composition that can apply clear to the skin after a minimal number of swipes or rubbing. Figure 3 illustrates a personal care product composition after two swipes of a finger. Figure 4 illustrates a personal care product composition after thre swipes of a finger. Figure 5 illustrates a personal care product composition after four swipes of a finger. Figure 6 illustrates a personal care product composition after five swipes of a finger. As seen through Figures 3-6, the whiteness of the personal care product composition may be gradually reduced through swipes of the finger. In Figure 6, whiteness that may form upon interaction of water and personal care product composition may have become substantially clear. Accordingly, in some embodiments, substantial removal of whiteness may be achieved after five swipes. A non-whitening composition, in some embodiments, may substantially clarify upon application to we skin - although some residue or residual whiteness may remain, a significant amount of whiteness has been removed through the application process.

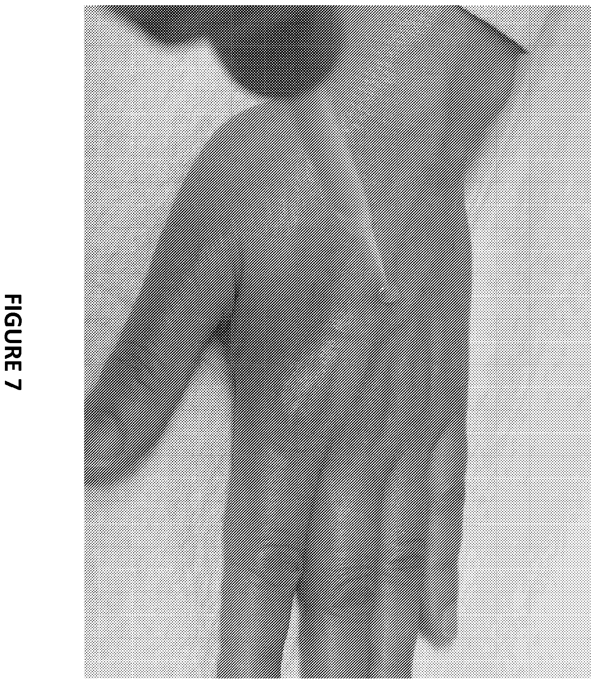

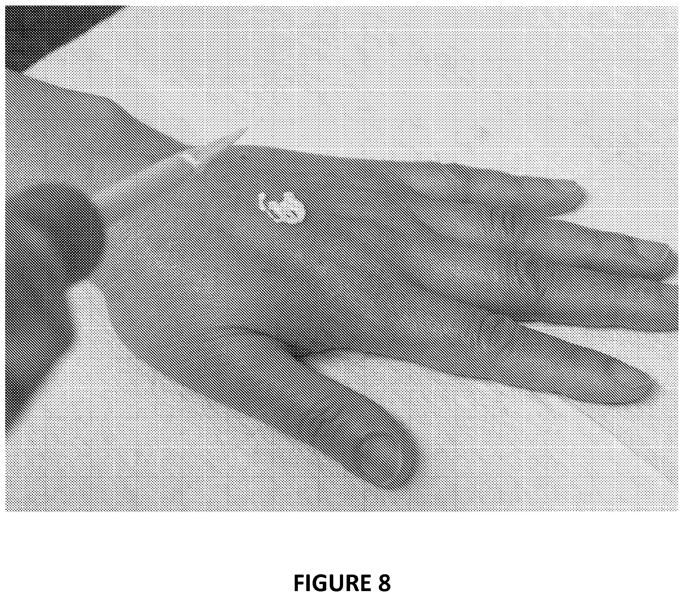

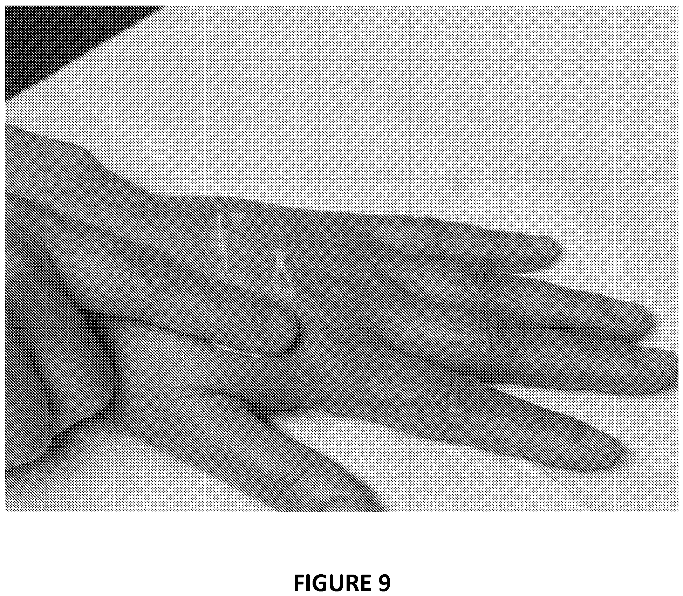

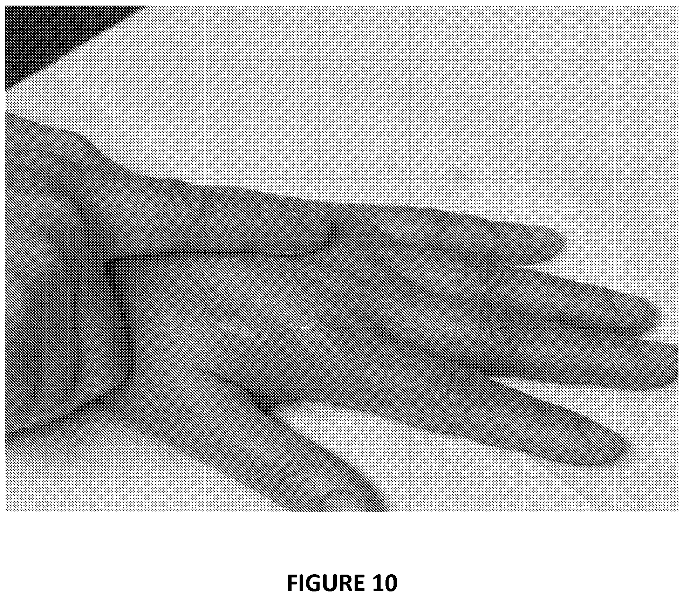

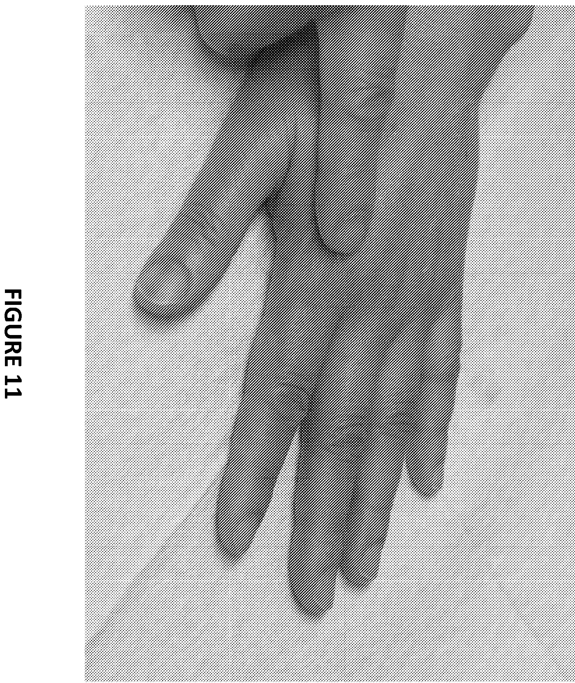

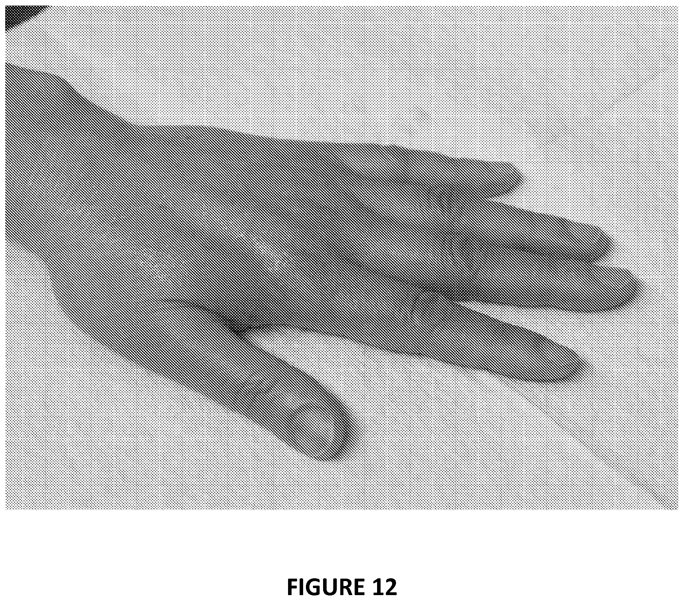

Figures 7-12 illustrate the application of another personal care product composition according to a specific example embodiment of the present disclosure. In Figure 7, 50 µl of deionized water is applied to the surface of a hand. In Figure 8, 50 mg of a personal care product composition according to a specific example embodiment of the present disclosure is applied to the same region where the water was applied. Figure 9 illustrates a personal care product composition after one swipe of a finger. Figure 10 illustrates a personal care product composition after two swipes of a finger. Figure 11 illustrates a personal care product composition after three swipes of a finger. As seen through Figures 9-11, the whiteness of the personal care product composition may be gradually reduced through swipes of the finger. In Figure 11, whiteness that may form upon interaction of water and personal care product composition may have become substantially clear. Accordingly, in some embodiments, substantial removal of whiteness may be achieved after as few as three swipes. Figure 12 illustrates a completed application of a personal care product. As seen in Figure 12, very little if any residue or whiteness remains. Thus, clear application of personal care product compositions according to embodiments of the present disclosure may be achieved in as little as three swipes of a finger.

## Claims

1. A method of formulating a personal care product composition that is stable when exposed to temperatures of about 60°C and/or about 5°C for a period of one week or more and that applies clear to wet skin and remains clear, the method comprising:
combining a discontinuous oil phase with a hydrophilic-lipophilic balance (HLB) emulsifying compound to form an oil phase mixture, wherein the HLB emulsifying compound is a liquid at room temperature and has a HLB less than about 5;
heating and mixing the oil phase mixture;
dispersing one or more carbomer polymers in a continuous water phase to form a water phase mixture;
combining and mixing the oil phase mixture and the water phase mixture to form a pre-emulsion;
adding a neutralizing base to the pre-emulsion to form a personal care product emulsion;
adding water to the personal care product emulsion; and
mixing the personal care product emulsion to form a homogenous composition, wherein, the homogenous composition comprises:
from 0.1% to 1% by weight of the HLB emulsifying compound,
a stabilizing, non-whitening amount of the one or more carbomer polymers, each carbomer polymer selected from the group consisting of a carbomer copolymer, a carbomer interpolymer, and a carbomer homopolymer, wherein none of the one or more carbomer polymers is emulsifying grade and wherein the total carbomer polymer concentration is less than 0.5% by weight of the composition, and
wherein, the homogenous composition is free of any other emulsifying compound.

2. A method according to claim 1 further comprising combining the discontinuous oil phase with one or more UV filters to form the oil phase mixture, wherein, the homogenous composition comprises up to 50% by weight of the one or more organic UV filters.

3. A method of formulating an oil-in-water personal care product composition that is stable when exposed to temperatures of about 60°C and/or about 5°Cfor a period of one week or more, the method comprising:
providing separately an oil phase and a water phase;
dispersing one or more carbomer polymers in either the oil phase or the water phase;
dispersing a hydrophilic-lipophilic balance (HLB) emulsifying compound in the oil phase, wherein the HLB emulsifying compound is a liquid at room temperature and has a HLB less than about 5;
combining the oil phase with the water phase to form a pre-emulsion; adding a neutralizing base to the pre-emulsion to form a personal care product emulsion;
adding water to the personal care product emulsion; and
mixing the personal care product emulsion to form a homogenous composition, wherein, the homogenous composition comprises:
from 0.1% to 1% by weight of the HLB emulsifying compound, a stabilizing amount of the one or more carbomer polymers, each carbomer polymer selected from the group consisting of a carbomer copolymer, a carbomer interpolymer, and a carbomer homopolymer, wherein none of the one or more carbomer polymers are emulsifying grade and wherein the total carbomer polymer concentration is less than 0.5% by weight of the composition, and
wherein, the homogenous composition is free of any other emulsifying compound.

4. A personal care product composition for topical application to human skin that applies clear to wet skin comprising:
a continuous water phase;
a discontinuous oil phase dispersed in the continuous water phase, the discontinuous oil phase optionally comprising one or more UV filters at a concentration of up to 50 wt.% of the total composition;
a hydrophilic-lipophilic balance (HLB) emulsifying compound at a concentration of 0.1 wt.% to 1 wt.% of the total composition, wherein the HLB emulsifying compound is a liquid at room temperature and has a HLB less than about 5, the composition lacking any other emulsifying compound;
a neutralizing base; and
a stabilizing, non-whitening amount of the one or more carbomer polymers, each carbomer polymer selected from the group consisting of a carbomer copolymer, a carbomer interpolymer, and a carbomer homopolymer, wherein none of the one or more carbomer polymers are emulsifying grade and wherein the total carbomer polymer concentration is less than 0.5% by weight of the composition, and
wherein the low HLB emulsifying compound is sorbitan oleate or glyceryl oleate.

5. A personal care product composition according to claim 4, wherein the continuous water phase constitutes a weight percent from 10% to 90% of the total composition, and/or wherein the discontinuous oil phase constitutes a weight percent from 10% to 80% of the total composition.

6. A personal care product composition according to claim 4 or claim 5, wherein the continuous water phase constitutes a weight percent of the total composition that is greater than the weight percent of the discontinuous oil phase.

7. A personal care product composition or method according to claim 6, wherein the HLB emulsifying compound is 0.1 wt.% to 0.8 wt.% of the total composition.

8. A personal care product composition or method according to claim 7, wherein the HLB emulsifying compound is sorbitan oleate at 0.2 wt.% to 0.4 wt.% of the total composition; or
a personal care product composition or method according to claim 8, wherein the HLB emulsifying compound is glyceryl oleate at 0.3 wt.% to 0.5 wt.% of the total composition.

9. A personal care product composition or method according to any preceding claim, wherein each of the UV filters are selected from the group of 2-ethylhexyl 2-cyano-3,3-diphenyl-2-propenoate, salicylic acid esters, ethylhexyl salicylate, homomenthyl salicylate, 3,3,5-trimethylcyclohexyl 2-hydroxybenzoate, p-methoxycinnamic acid esters, ethylhexyl methoxycinnamate, isoamyl methoxycinnamate, 1-(4-methoxyphenyl)-3-(4-tert-butylphenyl)propane-1,3-dione, butyl methoxydibenzoylmethane, (2-hydroxy-4-methoxyphenyl)-phenylmethanone, benzophenone-3, bemotrizinol, bis-ethylhexyloxyphenol methoxyphenyl triazone, methylene bis-benzotriazolyl, tetramethylbutylphenol, 4-methylbenzylidene camphor, diethylamino hydroxyl benzoyl hexyl benzoate, drometrizole trisiloxane, ethylhexyl triazone, diethylhexyl butamido triazone, terephthalyidene dicamphor sulfonic acid and salts thereof, and menthyl anthranilate.

10. A personal care product composition according to claim 4 or any preceding claim, wherein the composition is stable for at least about 1 month at about 5° C such that there is no change in the physical appearance of the personal care product composition; or
wherein the composition is stable for at least about 1 month at about 60° C such that there is no change in the physical appearance of the personal care product composition.

11. A personal care product composition according to claim 4 or any preceding claim, wherein the carbomer polymer comprises a carbomer homopolymer at 0.15 wt.% to 0.4 wt.% of the total composition; or
wherein the carbomer polymer comprises a carbomer homopolymer at 0.3 wt.% to 0.4 wt.% of the total composition; or
wherein the carbomer polymer comprises a carbomer interpolymer at 0.1 wt.% to 0.5 wt.% of the total composition; or
wherein the carbomer polymer comprises a carbomer interpolymer at 0.15 wt.% to 0.3 wt.% of the total composition; or
wherein the carbomer polymer comprises a carbomer copolymer at 0.1 wt.% to 0.5 wt.% of the total composition; or
wherein the carbomer polymer comprises a carbomer copolymer at 0.15 wt.% to 0.25 wt.% of the total composition; or
wherein the one or more carbomer polymers total 0.3 wt.% to 0.5 wt.% of the total composition.

12. A personal care product composition according to claim 4 or any preceding claim, wherein the one or more carbomer polymers comprises a carbomer homopolymer and a carbomer copolymer; or
wherein the one or more carbomer polymers comprises
a carbomer homopolymer at 0.15 wt.% of the total composition, and
a carbomer copolymer at 0.15 wt.% of the total composition.

13. A personal care product composition according to claim 4 or any preceding claim, wherein the one or more carbomer polymers comprises a carbomer homopolymer and a carbomer interpolymer; or
wherein the one or more carbomer polymers comprises
a carbomer homopolymer at 0.15 wt.% of the total composition, and
a carbomer interpolymer at 0.15 wt.% of the total composition.

14. A personal care product composition according to claim 4 or any preceding claim, wherein the continuous water phase comprises a water-soluble preservative, a humectant, a chelating agent, a pH adjuster, a buffer, a neutralizer, a colorant, a water-soluble biologically active compound or combinations thereof; or
wherein the continuous water phase comprises a glycol, a polyhydric alcohol or a glycol and a polyhydric alcohol; or
wherein the continuous water phase comprises a glycerin, a propylene glycol, a butylene glycol, a hexalene glycol or combinations thereof; or
wherein the continuous water phase comprises humectant at 0 wt.% to 10 wt.% of the total composition.

15. A personal care product composition according to claim 4 or any preceding claim, wherein the discontinuous oil phase further comprises an organic stabilizer, a stabilizer, a solvent, an emollient, a film-forming polymer, a SPF boosting compound, an SPF boosting polymer or combinations thereof.

16. A personal care product composition according to claim 15, wherein the stabilizer is selected from ethylhexyl methoxycrylene, diethylhexyl syringylidene malonate, diethylhexyl 2,6-naphthalate, polyester-8, polyester-25, undecylcrylene dimethicone, pentaerythrityl tetra-di-t-butyl hydroxyhydrocinnamate, octadecyl di-t-butyl-4-hydroxyhydrocinnamate, benzotriazolyl dodecyl p-cresol, Tris, citrate, sodium benzotriazolyl butylphenol sulfonate, bumetrizole, butylated hydroxyltoluene (BHT), butylated hydroxyanisole (BHA), tocopherol or combinations thereof; or
wherein the film-forming polymer is selected from acrylic homopolymers or copolymers with hydrophobic groups; dimethicone/acrylates dimethicone copolymers; hydrogenated dimer dilinoleyl/dimethylcarbonate copolymers; copolymers of vinylpyrrolidone and a long-chain alpha-olefin, vinylpyrrolidone/tricontanyl copolymer, VA/butyl maleate/isobornyl acrylate copolymer or combinations thereof; or
wherein the film-forming polymer comprises an acrylate dimethicone copolymer; or wherein the discontinuous oil phase further comprises a film-forming polymer at 1 wt.% to 3 wt.% of the total composition; or
wherein the discontinuous oil phase further comprises a fatty acid ester, a fatty alcohol ester, an ester of a Guerbet alcohol, a benzoic acid ester, an ester of a dibasic acid, an ester of a polyprotic acid, an ester of a polyhydric alcohol, a triglyceride, a polyethylene glycol, an ester of a polyethylene glycol, a polypropylene glycol, an ester of a polypropylene glycol, a polyester, a silicone, an organically-modified silicone, a hydrocarbon compound comprising a mineral oil, a paraffinic oil, a naphthenic oil, or an aromatic oil, a polymer derived from a polyisobutene, a polymer derived from a polypropylene, a C12-15 alkyl benzoate or combinations thereof.

17. A personal care product composition according to claim 4 or any preceding claim, wherein the personal care product composition comprises a viscosity of about 300 cP to about 115,000 cP.

18. A personal care product composition according to claim 4 or any preceding claim, wherein the personal care product is applied by swiping, rubbing, smoothing, massaging, coating, or otherwise working the composition on the skin for about 2 to about 5 seconds; or
wherein, the composition comprises a photoactive amount of the one or more organic UV filters up to 50% by weight of the total composition.

19. A method of applying a personal care product composition to a wet human skin surface having a natural appearance and color, the method comprising:
applying the personal care product composition to the wet human skin surface, the personal care product composition comprising:
a continuous water phase;
a discontinuous oil phase dispersed in the continuous water phase, the discontinuous oil phase comprising one or more UV filters at a concentration of at least 5 wt.% of the total composition;
a hydrophilic-lipophilic balance (HLB) emulsifying compound at a concentration of 0.1 wt.% to 1 wt.% of the total composition, wherein the HLB emulsifying compound is a liquid at room temperature and has a HLB of less than about 5, the composition lacking any other emulsifying compound;
a neutralizing base; and
a stabilizing, non-whitening amount of the one or more carbomer polymers, each carbomer polymer selected from the group consisting of a carbomer copolymer, a carbomer interpolymer, and a carbomer homopolymer, wherein none of the one or more carbomer polymers are emulsifying grade and wherein the total carbomer polymer concentration is less than 0.5% by weight of the composition,
wherein
the applying comprises swiping, rubbing, smoothing, massaging, coating, or otherwise working the composition on the skin,
the applying continues for less than about 5 seconds, and
the personal care product composition is clear no later than at the conclusion of the applying, and
the skin with the applied personal care product composition is shielded from exposure to at least one wavelength of light relative to skin without the personal care product, and optionally or preferably wherein the personal care product composition is clear throughout the applying.

## Patentansprüche

1. Verfahren zur Formulierung einer Körperpflegeproduktzusammensetzung, die bei Exposition gegenüber Temperaturen von etwa 60 °C und/oder etwa 5 °C für eine Dauer von einer Woche oder mehr stabil ist und die auf feuchte Haut klar aufträgt und klar bleibt, wobei das Verfahren umfasst:
Kombinieren einer diskontinuierlichen Ölphase mit einer hydrophil-lipophil-Gleichgewicht(HLB)-emulgierenden Verbindung, um ein Ölphasengemisch zu bilden, wobei die HLB-emulgierende Verbindung bei Raumtemperatur eine Flüssigkeit ist und ein HLB von weniger als etwa 5 aufweist;
Erwärmen und Mischen des Ölphasengemischs;
Dispergieren eines oder mehrerer Carbomerpolymere in einer kontinuierlichen Wasserphase, um ein Wasserphasengemisch zu bilden;
Kombinieren und Mischen des Ölphasengemischs und des Wasserphasengemischs, um eine Voremulsion zu bilden;
Zugeben einer neutralisierenden Base zu der Voremulsion, um eine Körperpflegeproduktemulsion zu bilden;
Zugeben von Wasser zu der Körperpflegeproduktemulsion; und
Mischen der Körperpflegeproduktemulsion, um eine homogene Zusammensetzung zu bilden, wobei die homogene Zusammensetzung umfasst:
von 0,1 Gew.-% bis 1 Gew.-% an der HLBemulgierenden Verbindung,
eine stabilisierende, nicht-aufhellende Menge des einen oder der mehreren Carbomerpolymere, wobei jedes Carbomerpolymer ausgewählt ist aus der Gruppe bestehend aus einem Carbomer-Copolymer, einem Carbomer-Interpolymer und einem Carbomer-Homopolymer, wobei keines von dem einen oder den mehreren Carbomerpolymeren eine emulgierende Sorte ist und wobei die Gesamt-Carbomerpolymerkonzentration weniger als 0,5 Gew.-% der Zusammensetzung beträgt, und
wobei die homogene Zusammensetzung frei von jeder anderen emulgierenden Verbindung ist.

2. Verfahren gemäß Anspruch 1, ferner umfassend Kombinieren der diskontinuierlichen Ölphase mit einem oder mehreren UV-Filtern, um das Ölphasengemisch zu bilden, wobei die homogene Zusammensetzung bis zu 50 Gew.-% an dem einen oder den mehreren UV-Filtern umfasst.

3. Verfahren zur Formulierung einer Öl-in-Wasser-Körperpflegeproduktzusammensetzung, die bei Exposition gegenüber Temperaturen von etwa 60 °C und/oder etwa 5 °C für eine Dauer von einer Woche oder mehr stabil ist, wobei das Verfahren umfasst:
getrenntes Bereitstellen einer Ölphase und einer Wasserphase;
Dispergieren eines oder mehrerer Carbomerpolymere entweder in der Ölphase oder in der Wasserphase;
Dispergieren einer hydrophil-lipophil-Gleichgewicht(HLB)-emulgierenden Verbindung in der Ölphase, wobei die HLB-emulgierende Verbindung bei Raumtemperatur eine Flüssigkeit ist und ein HLB von weniger als etwa 5 aufweist;
Kombinieren der Ölphase mit der Wasserphase, um eine Voremulsion zu bilden;
Zugeben einer neutralisierenden Base zu der Voremulsion, um eine Körperpflegeproduktemulsion zu bilden;
Zugeben von Wasser zu der Körperpflegeproduktemulsion; und
Mischen der Körperpflegeproduktemulsion, um eine homogene Zusammensetzung zu bilden, wobei die homogene Zusammensetzung umfasst:
von 0,1 Gew.-% bis 1 Gew.-% an der HLBemulgierenden Verbindung, eine stabilisierende Menge des einen oder der mehreren Carbomerpolymere, wobei jedes Carbomerpolymer ausgewählt ist aus der Gruppe bestehend aus einem Carbomer-Copolymer, einem Carbomer-Interpolymer und einem Carbomer-Homopolymer, wobei keines von dem einen oder den mehreren Carbomerpolymeren eine emulgierende Sorte ist und wobei die Gesamt-Carbomerpolymerkonzentration weniger als 0,5 Gew.-% der Zusammensetzung beträgt, und
wobei die homogene Zusammensetzung frei von jeder anderen emulgierenden Verbindung ist.

4. Körperpflegeproduktzusammensetzung zum topischen Aufbringen auf menschliche Haut, die klar auf feuchte Haut aufträgt, umfassend:
eine kontinuierliche Wasserphase;
eine diskontinuierliche Ölphase, die in der kontinuierlichen Wasserphase dispergiert ist, wobei die diskontinuierliche Ölphase gegebenenfalls einen oder mehrere UV-Filter mit einer Konzentration von bis zu 50 Gew.-% der gesamten Zusammensetzung umfasst;
eine hydrophil-lipophil-Gleichgewicht(HLB)-emulgierende Verbindung mit einer Konzentration von 0,1 Gew.-% bis 1 Gew.-% der gesamten Zusammensetzung, wobei die HLB-emulgierende Verbindung bei Raumtemperatur eine Flüssigkeit ist und ein HLB von weniger als etwa 5 aufweist, wobei die Zusammensetzung keine andere emulgierende Verbindung aufweist;
eine neutralisierende Base; und
eine stabilisierende, nicht-aufhellende Menge des einen oder der mehreren Carbomerpolymere, wobei jedes Carbomerpolymer ausgewählt ist aus der Gruppe bestehend aus einem Carbomer-Copolymer, einem Carbomer-Interpolymer und einem Carbomer-Homopolymer, wobei keines von dem einen oder den mehreren Carbomerpolymeren eine emulgierende Sorte ist und wobei die Gesamt-Carbomerpolymerkonzentration weniger als 0,5 Gew.-% der Zusammensetzung beträgt, und
wobei die niedrig-HLB-emulgierende Verbindung Sorbitanoleat oder Glyceryloleat ist.

5. Körperpflegeproduktzusammensetzung gemäß Anspruch 4, wobei die kontinuierliche Wasserphase einen Gewichtsprozentanteil von 10 % bis 90 % der gesamten Zusammensetzung bildet und/oder wobei die diskontinuierliche Ölphase einen Gewichtsprozentanteil von 10 % bis 80 % der gesamten Zusammensetzung bildet.

6. Körperpflegeproduktzusammensetzung gemäß Anspruch 4 oder Anspruch 5, wobei die kontinuierliche Wasserphase einen Gewichtsprozentanteil der gesamten Zusammensetzung bildet, der größer als der Gewichtsprozentanteil der diskontinuierlichen Ölphase ist.

7. Körperpflegeproduktzusammensetzung oder Verfahren gemäß Anspruch 6, wobei die HLB-emulgierende Verbindung 0,1 Gew.-% bis 0,8 Gew.-% der gesamten Zusammensetzung bildet.

8. Körperpflegeproduktzusammensetzung oder Verfahren gemäß Anspruch 7, wobei die HLB-emulgierende Verbindung Sorbitanoleat mit 0,2 Gew.-% bis 0,4 Gew.-% der gesamten Zusammensetzung ist; oder
Körperpflegeproduktzusammensetzung oder Verfahren gemäß Anspruch 8, wobei die HLB-emulgierende Verbindung Glyceryloleat mit 0,3 Gew.-% bis 0,5 Gew.-% der gesamten Zusammensetzung ist.

9. Körperpflegeproduktzusammensetzung oder Verfahren gemäß einem der vorstehenden Ansprüche, wobei jeder der UV-Filter ausgewählt ist aus der Gruppe von 2-Ethylhexyl-2-cyano-3,3-diphenyl-2-propenoat, Salicylsäureestern, Ethylhexylsalicylat, Homomenthylsalicylat, 3,3,5-Trimethylcyclohexyl-2-hydroxybenzoat, p-Methoxyzimtsäureestern, Ethylhexylmethoxycinnamat, Isoamylmethoxycinnamat, 1-(4-Methoxyphenyl)-3-(4-tert-butylphenyl)propan-1,3-dion, Butylmethoxydibenzoylmethan, (2-Hydroxy-4-methoxyphenyl)phenylmethanon, Benzophenon-3, Bemotrizinol, Bisethylhexyloxyphenolmethoxyphenyltriazon, Methylenbisbenzotriazolyl, Tetramethylbutylphenol, 4-Methylbenzylidencampher, Diethylaminohydroxybenzoylhexylbenzoat, Drometrizoltrisiloxan, Ethylhexyltriazon, Diethylhexylbutamidotriazon, Terephthalyidendicamphersulfonsäure und Salzen davon und Menthylanthranilat.

10. Körperpflegeproduktzusammensetzung gemäß Anspruch 4 oder einem der vorstehenden Ansprüche, wobei die Zusammensetzung bei etwa 5 °C für wenigstens etwa 1 Monat stabil ist, so dass keine Veränderung der äußeren Erscheinung der Körperpflegeproduktzusammensetzung auftritt; oder
wobei die Zusammensetzung bei etwa 60 °C für wenigstens etwa 1 Monat stabil ist, so dass keine Veränderung der äußeren Erscheinung der Körperpflegeproduktzusammensetzung auftritt.

11. Körperpflegeproduktzusammensetzung gemäß Anspruch 4 oder einem der vorstehenden Ansprüche, wobei das Carbomerpolymer ein Carbomer-Homopolymer mit 0,15 Gew.-% bis 0,4 Gew.-% der gesamten Zusammensetzung umfasst; oder
wobei das Carbomerpolymer ein Carbomer-Homopolymer mit 0,3 Gew.-% bis 0,4 Gew.-% der gesamten Zusammensetzung umfasst; oder
wobei das Carbomerpolymer ein Carbomer-Interpolymer mit 0,1 Gew.-% bis 0,5 Gew.-% der gesamten Zusammensetzung umfasst; oder
wobei das Carbomerpolymer ein Carbomer-Interpolymer mit 0,15 Gew.-% bis 0,3 Gew.-% der gesamten Zusammensetzung umfasst; oder
wobei das Carbomerpolymer ein Carbomer-Copolymer mit 0,1 Gew.-% bis 0,5 Gew.-% der gesamten Zusammensetzung umfasst; oder
wobei das Carbomerpolymer ein Carbomer-Copolymer mit 0,15 Gew.-% bis 0,25 Gew.-% der gesamten Zusammensetzung umfasst; oder
wobei das eine oder die mehreren Carbomerpolymere insgesamt 0,3 Gew.-% bis 0,5 Gew.-% der gesamten Zusammensetzung bilden.

12. Körperpflegeproduktzusammensetzung gemäß Anspruch 4 oder einem der vorstehenden Ansprüche, wobei das eine oder die mehreren Carbomerpolymere ein Carbomer-Homopolymer und ein Carbomer-Copolymer umfassen; oder
wobei das eine oder die mehreren Carbomerpolymere umfassen:
ein Carbomer-Homopolymer mit 0,15 Gew.-% der gesamten Zusammensetzung und
ein Carbomer-Copolymer mit 0,15 Gew.-% der gesamten Zusammensetzung.

13. Körperpflegeproduktzusammensetzung gemäß Anspruch 4 oder einem der vorstehenden Ansprüche, wobei das eine oder die mehreren Carbomerpolymere ein Carbomer-Homopolymer und ein Carbomer-Interpolymer umfassen; oder
wobei das eine oder die mehreren Carbomerpolymere umfassen:
ein Carbomer-Homopolymer mit 0,15 Gew.-% der gesamten Zusammensetzung und
ein Carbomer-Interpolymer mit 0,15 Gew.-% der gesamten Zusammensetzung.

14. Körperpflegeproduktzusammensetzung gemäß Anspruch 4 oder einem der vorstehenden Ansprüche, wobei die kontinuierliche Wasserphase ein wasserlösliches Konservierungsmittel, ein Feuchthaltemittel, einen Chelatbildner, ein pH-Einstellmittel, einen Puffer, ein Neutralisationsmittel, einen Farbstoff, einen wasserlöslichen biologischen Wirkstoff oder eine Kombination davon umfasst; oder
wobei die kontinuierliche Wasserphase ein Glycol, einen polyhydrischen Alkohol oder ein Glycol und einen polyhydrischen Alkohol umfasst; oder
wobei die kontinuierliche Wasserphase ein Glycerin, ein Propylenglycol, ein Butylenglycol, ein Hexalenglycol oder Kombinationen davon umfasst; oder
wobei die kontinuierliche Wasserphase Feuchthaltemittel mit 0 Gew.-% bis 10 Gew.-% der gesamten Zusammensetzung umfasst.

15. Körperpflegeproduktzusammensetzung gemäß Anspruch 4 oder einem der vorstehenden Ansprüche, wobei die diskontinuierliche Ölphase ferner einen organischen Stabilisator, einen Stabilisator, ein Lösungsmittel, einen Weichmacher, ein filmbildendes Polymer, eine SPF-Verstärkerverbindung, ein SPF-Verstärkerpolymer oder Kombinationen davon umfasst.

16. Körperpflegeproduktzusammensetzung gemäß Anspruch 15, wobei der Stabilisator ausgewählt ist aus Ethylhexylmethoxycrylen, Diethylhexylsyringylidenmalonat, Diethylhexyl-2,6-naphthalat, Polyester-8, Polyester-25, Undecylcrylendimethicon, Pentaerythrityltetradi-t-butylhydroxyhydrocinnamat, Octadecyldi-t-butyl-4-hydroxyhydrocinnamat, Benzotriazolyldodecyl-p-cresol, Tris, Citrat, Natriumbenzotriazolylbutylphenolsulfonat, Bumetrizol, butyliertem Hydroxyltoluol (BHT), butyliertem Hydroxyanisol (BHA), Tocopherol oder Kombinationen davon; oder
wobei das filmbildende Polymer ausgewählt ist aus Acryl-Homopolymeren oder -Copolymeren mit hydrophoben Gruppen; Dimethicon/Acrylat-Dimethicon-Copolymeren; hydrierten Dimer-Dilinoleyl/Dimethylcarbonat-Copolymeren; Copolymeren von Vinylpyrrolidon und einem langkettigen alpha-Olefin, Vinylpyrrolidon/Tricontanyl-Copolymer, VA/Butylmaleat/Isobornylacrylat-Copolymer oder Kombinationen davon; oder
wobei das filmbildende Polymer ein Acrylat-Dimethicon-Copolymer umfasst; oder
wobei die diskontinuierliche Ölphase ferner ein filmbildendes Polymer mit 1 Gew.-% bis 3 Gew.-% der gesamten Zusammensetzung umfasst; oder
wobei die diskontinuierliche Ölphase ferner einen Fettsäureester, einen Fettalkoholester, einen Ester eines Guerbet-Alkohols, einen Benzoesäureester, einen Ester einer zweibasigen Säure, einen Ester einer polyprotischen Säure, einen Ester eines polyhydrischen Alkohols, ein Triglycerid, ein Polyethylenglycol, einen Ester eines Polyethylenglycols, ein Polypropylenglycol, einen Ester eines Polypropylenglycols, einen Polyester, ein Silicon, ein organisch modifiziertes Silicon, eine Kohlenwasserstoffverbindung, die ein Mineralöl, ein Paraffinöl, ein Naphthenöl oder ein aromatisches Öl umfasst, ein von einem Polyisobuten abgeleitetes Polymer, ein von einem Polypropylen abgeleitetes Polymer, ein C12-15-Alkylbenzoat oder eine Kombination davon umfasst.

17. Körperpflegeproduktzusammensetzung gemäß Anspruch 4 oder einem der vorstehenden Ansprüche, wobei die Körperpflegeproduktzusammensetzung eine Viskosität von etwa 300 cP bis etwa 115.000 cP umfasst.

18. Körperpflegeproduktzusammensetzung gemäß Anspruch 4 oder einem der vorstehenden Ansprüche, wobei das Körperpflegeprodukt durch Wischen, Reiben, Glätten, Massieren, Überziehen oder sonstiges Verarbeiten der Zusammensetzung auf der Haut für etwa 2 bis etwa 5 Sekunden aufgebracht wird; oder
wobei die Zusammensetzung eine photoaktive Menge des einen oder der mehreren organischen UV-Filter bis zu 50 Gew.-% der gesamten Zusammensetzung umfasst.

19. Verfahren zum Aufbringen einer Körperpflegeproduktzusammensetzung auf eine feuchte menschliche Hautoberfläche, die ein natürliches Aussehen und eine natürliche Farbe aufweist, wobei das Verfahren umfasst:
Aufbringen der Körperpflegeproduktzusammensetzung auf die feuchte menschliche Hautoberfläche,
wobei die Körperpflegeproduktzusammensetzung umfasst:
eine kontinuierliche Wasserphase;
eine diskontinuierliche Ölphase, die in der kontinuierlichen Wasserphase dispergiert ist, wobei die diskontinuierliche Ölphase einen oder mehrere UV-Filter mit einer Konzentration von wenigstens 5 Gew.-% der gesamten Zusammensetzung umfasst;
eine hydrophil-lipophil-Gleichgewicht(HLB)-emulgierende Verbindung mit einer Konzentration von 0,1 Gew.-% bis 1 Gew.-% der gesamten Zusammensetzung, wobei die HLB-emulgierende Verbindung bei Raumtemperatur eine Flüssigkeit ist und ein HLB von weniger als etwa 5 aufweist, wobei die Zusammensetzung keine andere emulgierende Verbindung aufweist;
eine neutralisierende Base; und
eine stabilisierende, nicht-aufhellende Menge des einen oder der mehreren Carbomerpolymere, wobei jedes Carbomerpolymer ausgewählt ist aus der Gruppe bestehend aus einem Carbomer-Copolymer, einem Carbomer-Interpolymer und einem Carbomer-Homopolymer, wobei keines von dem einen oder den mehreren Carbomerpolymeren eine emulgierende Sorte ist und wobei die Gesamt-Carbomerpolymerkonzentration weniger als 0,5 Gew.-% der Zusammensetzung beträgt,
wobei
das Aufbringen Wischen, Reiben, Glätten, Massieren, Überziehen oder sonstiges Verarbeiten der Zusammensetzung auf der Haut umfasst,
das Aufbringen weniger als etwa 5 Sekunden andauert und
die Körperpflegeproduktzusammensetzung nicht später als am Ende des Aufbringens klar ist und
die Haut mit der aufgebrachten Körperpflegeproduktzusammensetzung vor Exposition gegenüber wenigstens einer Lichtwellenlänge im Vergleich zu Haut ohne das Körperpflegeprodukt abgeschirmt ist und gegebenenfalls oder vorzugsweise wobei die Körperpflegeproduktzusammensetzung während des gesamten Aufbringens klar ist.

## Revendications

1. Procédé de formulation d'une composition de produit de soin personnel qui est stable lorsqu'elle est exposée à des températures d'environ 60 °C et/ou environ 5 °C pendant une durée d'une semaine ou plus et qui est transparente lorsqu'elle est appliquée sur la peau humide et reste transparente, le procédé comprenant :
la combinaison d'une phase huileuse discontinue avec un composé émulsifiant d'équilibre hydrophile-lipophile (HLB) pour former un mélange de phase huileuse, dans lequel le composé émulsifiant HLB est un liquide à température ambiante et présente un HLB inférieur à environ 5 ;
le chauffage et le mélange du mélange de phase huileuse ;
la dispersion d'un ou plusieurs polymères de carbomère dans une phase aqueuse continue pour former un mélange de phase aqueuse ;
la combinaison et le mélange du mélange de phase huileuse et du mélange de phase aqueuse pour former une préémulsion ;
l'ajout d'une base neutralisante à la préémulsion pour former une émulsion de produit de soin personnel ;
l'ajout d'eau à l'émulsion de produit de soin personnel ; et
le mélange de l'émulsion de produit de soin personnel pour former une composition homogène, dans lequel, la composition homogène comprend :
de 0,1 % à 1 % en poids du composé émulsifiant HLB,
une quantité stabilisante, non blanchissante des un ou plusieurs polymères de carbomère, chaque polymère de carbomère étant choisi dans le groupe constitué d'un copolymère de carbomère, un interpolymère de carbomère et un homopolymère de carbomère, dans lequel aucun des un ou plusieurs polymères de carbomère n'est de qualité émulsifiante et dans lequel la concentration de polymère de carbomère totale est inférieure à 0,5 % en poids de la composition, et
dans lequel, la composition homogène est exempte de tout autre composé émulsifiant.

2. Procédé selon la revendication 1, comprenant en outre la combinaison de la phase huileuse discontinue avec un ou plusieurs filtres UV pour former le mélange de phase huileuse,
dans lequel, la composition homogène comprend jusqu'à 50 % en poids des un ou plusieurs filtres UV organiques.

3. Procédé de formulation d'une composition de produit de soin personnel huile dans eau qui est stable lorsqu'elle est exposée à des températures d'environ 60 °C et/ou environ 5 °C pendant une durée d'une semaine ou plus, le procédé comprenant :
la fourniture, séparément, d'une phase huileuse et d'une phase aqueuse ;
la dispersion d'un ou plusieurs polymères de carbomère dans la phase huileuse ou la phase aqueuse ;
la dispersion d'un composé émulsifiant d'équilibre hydrophile-lipophile (HLB) dans la phase huileuse, dans lequel le composé émulsifiant HLB est un liquide à température ambiante et présente un HLB inférieur à environ 5 ;
la combinaison de la phase huileuse avec la phase aqueuse pour former une préémulsion ; l'ajout d'une base neutralisant à la préémulsion pour former une émulsion de produit de soin personnel ;
l'ajout d'eau à l'émulsion de produit de soin personnel ; et
le mélange de l'émulsion de produit de soin personnel pour former une composition homogène, dans lequel, la composition homogène comprend :
de 0,1 % à 1 % en poids du composé émulsifiant HLB, une quantité stabilisante des un ou plusieurs polymères de carbomère, chaque polymère de carbomère étant choisi dans le groupe constitué d'un copolymère de carbomère, un interpolymère de carbomère et un homopolymère de carbomère, dans lequel aucun des un ou plusieurs polymères de carbomère n'est de qualité émulsifiante et dans lequel la concentration de polymère de carbomère totale est inférieure à 0,5 % en poids de la composition, et
dans lequel, la composition homogène est exempte de tout autre composé émulsifiant.

4. Composition de produit de soin personnel pour application topique sur la peau humaine qui est transparente lorsqu'elle est appliquée sur la peau humide comprenant :
une phase aqueuse continue ;
une phase huileuse discontinue dispersée dans la phase aqueuse continue, la phase huileuse discontinue comprenant facultativement un ou plusieurs filtres UV à une concentration de jusqu'à 50 % en poids de la composition totale ;
un composé émulsifiant d'équilibre hydrophile-lipophile (HLB) à une concentration de 0,1 % en poids à 1 % en poids de la composition totale, dans laquelle le composé émulsifiant HLB est un liquide à température ambiante et présente un HLB inférieur à environ 5, la composition ne comportant aucun autre composé émulsifiant ;
une base neutralisante ; et
une quantité stabilisante, non blanchissante des un ou plusieurs polymères de carbomère, chaque polymère de carbomère étant choisi dans le groupe constitué d'un copolymère de carbomère, un interpolymère de carbomère et un homopolymère de carbomère, dans laquelle aucun des un ou plusieurs polymères de carbomère n'est de qualité émulsifiante et dans laquelle la concentration de polymère de carbomère totale est inférieure à 0,5 % en poids de la composition, et
dans laquelle le composé émulsifiant à HLB faible est l'oléate de sorbitane ou l'oléate de glycéryle.

5. Composition de produit de soin personnel selon la revendication 4, dans laquelle la phase aqueuse continue constitue un pourcentage en poids de 10 % à 90 % de la composition totale, et/ou dans laquelle la phase huileuse discontinue constitue un pourcentage en poids de 10 % à 80 % de la composition totale.

6. Composition de produit de soin personnel selon la revendication 4 ou la revendication 5, dans laquelle la phase aqueuse continue constitue un pourcentage en poids de la composition totale qui est supérieur au pourcentage en poids de la phase huileuse discontinue.

7. Composition de produit de soin personnel ou procédé selon la revendication 6, dans laquelle le composé émulsifiant HLB est de 0,1 % en poids à 0,8 % en poids de la composition totale.

8. Composition de produit de soin personnel ou procédé selon la revendication 7, dans lequel le composé émulsifiant HLB est l'oléate de sorbitane à 0,2 % en poids à 0,4 % en poids de la composition totale ; ou
composition de produit de soin personnel ou procédé selon la revendication 8, dans lequel le composé émulsifiant HLB est l'oléate de glycéryle à 0,3 % en poids à 0,5 % en poids de la composition totale.

9. Composition de produit de soin personnel ou procédé selon l'une quelconque des revendications précédentes, dans lequel chacun des filtres UV est choisi dans le groupe des 2-cyano-3,3-diphényl-2-propénoate de 2-éthylhexyle, esters d'acide salicylique, salicylate d'éthylhexyle, salicylate d'homomenthyle, 2-hydroxybenzoate de 3,3,5-triméthylcyclohexyle, esters d'acide p-méthoxycinnamique, méthoxycinnamate d'éthylhexyle, méthoxycinnamate d'isoamyle, 1-(4-méthoxyphényl)-3-(4-tert-butylphényl)propane-1,3-dione, butyl-méthoxydibenzoylméthane, (2-hydroxy-4-méthoxyphényl)-phénylméthanone, benzophénone-3, bémotrizinol, bis-éthylhexyloxyphénol-méthoxyphényl-triazone, méthylène-bis-benzotriazolyle, tétraméthylbutylphénol, 4-méthylbenzylidène-camphre, benzoate de diéthylaminohydroxylbenzoylhexyle, drométrizoletrisiloxane, éthylhexyltriazone, diéthylhexylbutamidotriazone, acide téréphtalylidènedicamphre-sulfonique et sels de celui-ci, et anthranilate de menthyle.

10. Composition de produit de soin personnel selon la revendication 4 ou l'une quelconque des revendications précédentes, la composition étant stable pendant au moins environ 1 mois à environ 5 °C de sorte qu'il n'y ait pas de changement d'aspect physique de la composition de produit de soin personnel ; ou
la composition étant stable pendant au moins environ 1 mois à environ 60 °C de sorte qu'il n'y ait pas de changement de l'aspect physique de la composition de produit de soin personnel.

11. Composition de produit de soin personnel selon la revendication 4 ou l'une quelconque des revendications précédentes, dans laquelle le polymère de carbomère comprend un homopolymère de carbomère à 0,15 % en poids à 0,4 % en poids de la composition totale ; ou
dans laquelle le polymère de carbomère comprend un homopolymère de carbomère à 0,3 % en poids à 0,4 % en poids de la composition totale ; ou
dans laquelle le polymère de carbomère comprend un interpolymère de carbomère à 0,1 % en poids à 0,5 % en poids de la composition totale ; ou
dans laquelle le polymère de carbomère comprend un interpolymère de carbomère à 0,15 % en poids à 0,3 % en poids de la composition totale ; ou
dans laquelle le polymère de carbomère comprend un copolymère de carbomère à 0,1 % en poids à 0,5 % en poids de la composition totale ; ou
dans laquelle le polymère de carbomère comprend un copolymère de carbomère à 0,15 % en poids à 0,25 % en poids de la composition totale ; ou
dans laquelle les un ou plusieurs polymères de carbomère constituent au total 0,3 % en poids à 0,5 % en poids de la composition totale.

12. Composition de produit de soin personnel selon la revendication 4 ou l'une quelconque des revendications précédentes, dans laquelle les un ou plusieurs polymères de carbomère comprennent un homopolymère de carbomère et un copolymère de carbomère ; ou dans laquelle les un ou plusieurs polymères de carbomère comprennent un homopolymère de carbomère à 0,15 % en poids de la composition totale, et un copolymère de carbomère à 0,15 % en poids de la composition totale.

13. Composition de produit de soin personnel selon la revendication 4 ou l'une quelconque des revendications précédentes, dans laquelle les un ou plusieurs polymères de carbomère comprennent un homopolymère de carbomère et un interpolymère de carbomère ; ou dans laquelle les un ou plusieurs polymères de carbomère comprennent un homopolymère de carbomère à 0,15 % en poids de la composition totale, et un interpolymère de carbomère à 0,15 % en poids de la composition totale.

14. Composition de produit de soin personnel selon la revendication 4 ou l'une quelconque des revendications précédentes, dans laquelle la phase aqueuse continue comprend un conservateur hydrosoluble, un humectant, un agent chélateur, un agent d'ajustement du pH, un tampon, un agent neutralisant, un colorant, un composé biologiquement actif hydrosoluble ou des combinaisons de ceux-ci ; ou
dans laquelle la phase aqueuse continue comprend un glycol, un alcool polyhydrique ou un glycol et un alcool polyhydrique ; ou
dans laquelle la phase aqueuse continue comprend une glycérine, un propylène glycol, un butylène glycol, un hexalène glycol ou des combinaisons de ceux-ci ; ou dans laquelle la phase aqueuse continue comprend un humectant à 0 % en poids à 10 % en poids de la composition totale.

15. Composition de produit de soin personnel selon la revendication 4 ou l'une quelconque des revendications précédentes, dans laquelle la phase huileuse discontinue comprend en outre un stabilisant organique, un stabilisant, un solvant, un émollient, un polymère filmogène, un composé augmentant SPF, un polymère augmentant SPF ou des combinaisons de ceux-ci.

16. Composition de produit de soin personnel selon la revendication 15, dans laquelle le stabilisant est choisi parmi les éthylhexyl-méthoxycrylène, malonate de diéthylhexyl-syringylidène, 2,6-naphtalate de diéthylhexyle, polyester-8, polyester-25, undecylcrylène-diméthicone, tétra-di-t-butyl-hydroxyhydrocinnamate de pentaérythrityle, di-t-butyl-4-hydroxyhydrocinnamate d'octadécyle, benzotriazolyl-dodécyl-p-crésol, Tris, citrate, benzotriazolylbutylphénolsulfonate de sodium, bumétrizole, hydroxyltoluène butylé (BHT), hydroxyanisole butylé (BHA), tocophérol ou des combinaisons de ceux-ci ; ou
dans laquelle le polymère filmogène est choisi parmi des homopolymères ou copolymères acryliques comportant des groupes hydrophobes ; des copolymères de diméthicone/acrylates diméthicone ; des copolymères de dimère de dilinoléyle hydrogéné/carbonate de diméthyle ; des copolymères de vinylpyrrolidone et d'une alpha-oléfine à chaîne longue, un copolymère de vinylpyrrolidone/tricontanyle, un copolymère de VA/maléate de butyle/acrylate d'isobornyle ou des combinaisons de ceux-ci ; ou
dans laquelle le polymère filmogène comprend un copolymère d'acrylate-diméthicone ; ou
dans laquelle la phase huileuse discontinue comprend en outre un polymère filmogène à 1 % en poids à 3 % en poids de la composition totale ; ou
dans laquelle la phase huileuse discontinue comprend en outre un ester d'acide gras, un ester d'alcool gras, un ester d'un alcool de Guerbet, un ester d'acide benzoïque, un ester d'un acide dibasique, un ester d'un acide polyprotique, un ester d'un alcool polyhydrique, un triglycéride, un polyéthylène glycol, un ester d'un polyéthylène glycol, un polypropylène glycol, un ester d'un polypropylène glycol, un polyester, un silicone, un silicone à modification organique, un composé hydrocarbure comprenant une huile minérale, une huile paraffinique, une huile naphténique, ou une huile aromatique, un polymère dérivé d'un polyisobutène, un polymère dérivé d'un polypropylène, un benzoate d'alkyle en C12-15 ou des combinaisons de ceux-ci.

17. Composition de produit de soin personnel selon la revendication 4 ou l'une quelconque des revendications précédentes, la composition de produit de soin personnel présentant une viscosité d'environ 300 cP à environ 115 000 cP.

18. Composition de produit de soin personnel selon la revendication 4 ou l'une quelconque des revendications précédentes, le produit de soin personnel est appliqué par tapotement, frottement, lissage, massage, revêtement, ou en travaillant autrement la composition sur la peau pendant environ 2 à environ 5 secondes ; ou
la composition comprenant une quantité photoactive des un ou plusieurs filtres UV organiques allant jusqu'à 50 % en poids de la composition totale.

19. Procédé d'application d'une composition de produit de soin personnel sur une surface de peau humaine humide ayant un aspect et une couleur naturels, le procédé comprenant :
l'application de la composition de produit de soin personnel sur la surface de peau humaine humide, la composition de produit de soin personnel comprenant :
une phase aqueuse continue ;
une phase huileuse discontinue dispersée dans la phase aqueuse continue, la phase huileuse discontinue comprenant un ou plusieurs filtres UV à une concentration d'au moins 5 % en poids de la composition totale ;
un composé émulsifiant d'équilibre hydrophile-lipophile (HLB) à une concentration de 0,1 % en poids à 1 % en poids de la composition totale, dans lequel le composé émulsifiant HLB est un liquide à température ambiante et présente un HLB inférieur à environ 5, la composition ne comportant aucun autre composé émulsifiant ;
une base neutralisante ; et
une quantité stabilisante, non blanchissante des un ou plusieurs polymères de carbomère, chaque polymère de carbomère étant choisi dans le groupe constitué d'un copolymère de carbomère, un interpolymère de carbomère et un homopolymère de carbomère, dans lequel aucun des un ou plusieurs polymères de carbomère n'est de qualité émulsifiante et dans lequel la concentration de polymère de carbomère totale est inférieure à 0,5 % en poids de la composition,
dans lequel
l'application comprend le tapotement, le frottement, le lissage, le massage, le revêtement, ou le travail autrement de la composition sur la peau,
l'application continue pendant environ 5 secondes, et
la composition de produit de soin personnel est transparente pas plus tard qu'à la fin de l'application, et
la peau sur laquelle la composition de produit de soin personnel est appliquée est protégée contre l'exposition à au moins une longueur d'onde de lumière par rapport à la peau sans le produit de soin personnel et, facultativement ou de préférence, dans lequel la composition de produit de soin personnel est transparente tout au long de l'application.
